# EUROPÄISCHE PATENTANMELDUNG

(11) **EP 1 607 482 A1**
(43) Veröffentlichungstag der Anmeldung: **21.12.2005**
(21) Anmeldenummer: 04014124.4
(22) Anmeldetag: 16.06.2004
(51) Int. Cl.: C12N 15/82, C12N 15/64, C12N 15/65

(54) **RNAi-basierte Verfahren zur Selektion von transfizierten eukaryontischen Zellen**

(71) Anmelder: GBF-GESELLSCHAFT FÜR BIOTECHNOLOGISCHE FORSCHUNG MBH, 38124 Braunschweig (DE)
(72) Erfinder: Hafner, Martin, 38300 Wolfenbüttel (DE); Müller, Werner, 38124 Braunschweig (DE); Nawrath, Karina, 38112 Braunschweig (DE)
(74) Vertreter: Vossius & Partner

(57) **Zusammenfassung**

Die Erfindung betrifft ein Verfahren zur Herstellung einer durch Inaktivierung oder Reduzierung einer endogenen Genfunktion selektionierbaren eukaryontischen Zelle, umfassend die Schritte (a) der Einführung einer oder mehrerer Vektoren in die Zelle und (b) der Expression einer von dem einen oder den mehreren Vektoren kodierten siRNA und vorzugsweise shRNA, die gegen ein endogenes selektionierbares Gen gerichtet ist und dieses inaktiviert, wobei die siRNA oder shRNA das Transkriptionsprodukt einer RNAi-Selektionskassette ist, wobei die Selektionskassette einen zumindest 19 Nukleotide langen Abschnitt der transkribierten Region des Gens umfasst, der funktionell mit einem Promoter und einem Transkriptionsterminationssignal verbunden ist. Darüber hinaus betrifft die Erfindung eine eukaryontische Zelle, umfassend eine RNAi-Selektionskassette die gegen ein endogenes selektionierbares Gen gerichtet ist, und dessen Funktion inaktiviert; wobei die RNAi-Selektionskassette einen zumindest 19 Nukleotide langen Abschnitt des Gens umfasst, der funktionell mit einem Promotor und einem Transkriptionsterminationssignal verbunden ist. Weiterhin betrifft die Erfindung Verfahren zur Herstellung eines transgenen Säugers, umfassend die Schritte: (a) Injektion der erfindungsgemäßen embryonalen Stammzelle oder einer nach dem erfindungsgemäßen Verfahren selektionierten embryonalen Stammzelle in Blastocysten eines Säugers, (b) Übertragen der Blastocysten in die Gebärmutter eines Säugers, und (c) Austragen des transgenen Säugers. Schliesslich betrifft die Erfindung ein Verfahren zur Herstellung einer transgenen Pflanze.

## Beschreibung

Die Erfindung betrifft ein Verfahren zur Herstellung einer durch Inaktivierung oder Reduzierung einer endogenen Genfunktion selektionierbaren eukaryontischen Zelle, umfassend die Schritte (a) der Einführung einer oder mehrerer Vektoren in die Zelle und (b) der Expression einer von dem einen oder den mehreren Vektoren kodierten siRNA und vorzugsweise shRNA, die gegen ein endogenes selektionierbares Gen gerichtet ist und dieses inaktiviert, wobei die siRNA oder shRNA das Transkriptionsprodukt einer RNAi-Selektionskassette ist, wobei die Selektionskassette einen zumindest 19 Nukleotide langen Abschnitt der transkribierten Region des Gens umfasst, der funktionell mit einem Promoter und einem Transkriptionsterminationssignal verbunden ist. Darüber hinaus betrifft die Erfindung eine eukaryontische Zelle, umfassend eine RNAi-Selektionskassette die gegen ein endogenes selektionierbares Gen gerichtet ist, und dessen Funktion inaktiviert; wobei die RNAi-Selektionskassette einen zumindest 19 Nukleotide langen Abschnitt des Gens umfasst, der funktionell mit einem Promotor und einem Transkriptionsterminationssignal verbunden ist. Weiterhin betrifft die Erfindung Verfahren zur Herstellung eines transgenen Säugers, umfassend die Schritte: (a) Injektion der erfindungsgemäßen embryonalen Stammzelle oder einer nach dem erfindungsgemäßen Verfahren selektionierten embryonalen Stammzelle in Blastocysten eines Säugers, (b) Übertragen der Blastocysten in die Gebärmutter eines Säugers, und (c) Austragen des transgenen Säugers. Schliesslich betrifft die Erfindung ein Verfahren zur Herstellung einer transgenen Pflanze.

In der Beschreibung sind eine Reihe von Dokumenten zitiert. Der Offenbarungsgehalt dieser Dokumente inklusive von Gebrauchsanweisungen von Herstellern ist hiermit per Referenz inkorporiert.

Die gentechnische Einführung genetischer Veränderungen sind sowohl in Pro- als auch in Eukaryonten seltene Ereignisse, weshalb geeignete Selektionsverfahren zur Anreicherung von Klonen, in denen das gewünschte Ereignis stattgefunden hat, benötigt werden. Grundsätzlich lassen sich zwei Möglichkeiten unterscheiden: Positive Selektion kann angewendet werden um solche Klone zu selektionieren, die eine bestimmte Eigenschaft besitzen. Im Gegensatz dazu reichert man mit negativer Selektion Zellen an, denen eine bestimmte Eigenschaft fehlt. Für positive Selektion kommen zum einen Selektionskassetten in Frage, die ein Produkt kodieren, das ein Überleben in einem Mangelmedium ermöglicht. Alternativ können beispielsweise Expressionskassetten zur positiven Selektion eingesetzt, deren Produkt ein im Medium enthaltenes Toxin inaktivieren und so ein Überleben des gentechnisch veränderten Organismus ermöglichen. Im Falle der negativen Selektion verhindert das Produkt an sich ein Überleben des Organismus oder das von der Selektionskassette exprimierte Produkt wandelt beispielsweise eine ungiftige Vorstufe in ein Toxin um, das den betreffenden Organismus dann tötet. Mit einigen Enzymen kann in Abhängigkeit von den jeweils im Medium vorhandenen Substanzen sowohl positiv als auch negativ selektioniert werden. Z.B. sind entsprechende Substanzen für Enzyme des Nukleotidstoffwechsel bekannt. Dabei stellt sich allerdings das Problem, dass die entsprechenden Enzyme in eukaryontischen Zellen normalerweise endogen exprimiert werden. Um entsprechende Selektionskassetten anwenden zu können, müssten also zunächst Zellen erzeugt werden, denen jeweils eines der entsprechenden Enzyme (HPRT, APRT, TK, DHFR usw.) fehlt. Tatsächlich finden solche Zellen in der Mutationsforschung bereits seit Jahren Anwendung. HPRT negative ES-Zellen werden außerdem bereits für die homologe Rekombination verwendet, wobei die dabei benutzten targeting-Konstrukte eine HPRT-Expressionskassette zur positiven Selektion enthalten. HPRT bietet den Vorteil, daß mittels HAT-(Hypoxanthin-Aminopterin-Thymidin)-Medium für und durch die Verwendung von 6-Thioguanosin oder 8-Azaguanosin gegen den Besitz eines funktionellen HPRT-Gens selektioniert werden kann.

Konventionelle Selektionssysteme basieren im wesentlichen auf den selektierbaren Eigenschaften, die von Proteinen vermittelt werden, die durch in die Zelle eingebrachte Nukleinsäuresequenzen kodiert werden. Diese fremden, d.h. exogenen Proteine haben zumeist allergenen Charakter und können in Säugern, insbesondere beim Menschen eine heftige Immunantwort induzieren. Dadurch ist der Einsatz solcher Selektionssysteme zum Beispiel in Vektoren zur Gentherapie oder bei der Herstellung transgener Pflanzen begrenzt. Darüber hinaus besteht bei der Verwendung von Proteinen, die durch Resistenzgene kodiert werden, häufig die Gefahr der Resistenzbildung in Prokaryonten.

Aufgrund der steigenden Bedeutung der Gentherapie bei der Behandlung von Krankheiten und angesichts des zunehmenden Einsatzes von transgenen Pflanzen in der Nahrungsmittelproduktion gibt es einen dringenden Bedarf an Selektionsverfahren, die auf die Expression von fremden Proteinen in den gentechnisch veränderten Zellen verzichten.

Der vorliegenden Erfindung liegt daher die Aufgabe zugrunde, ein für die Umwelt sicheres Selektionsverfahren bereitzustellen, dass weder Resistenzen bei Prokaryonten vermitteln kann, noch eine allergene oder immunstimulierende Wirkung insbesondere für den menschliche Organismus hat. Diese Aufgabe wird erfindungsgemäß durch die Bereitstellung der in den Patentansprüchen charakterisierten Ausführungsformen gelöst.

Somit betrifft die Erfindung ein Verfahren zur Herstellung einer durch Inaktivierung oder Reduzierung einer endogenen Genfunktion selektionierbaren eukaryontischen Zelle, umfassend die Schritte (a) der Einführung einer oder mehrerer Vektoren in die Zelle und (b) der Expression einer von dem einen oder den mehreren Vektoren kodierten siRNA und vorzugsweise shRNA, die gegen ein endogenes selektionierbares Gen gerichtet ist und dieses inaktiviert, wobei die siRNA oder shRNA das Transkriptionsprodukt einer RNAi-Selektionskassette ist, wobei die Selektionskassette einen zumindest 19 Nukleotide langen Abschnitt der transkribierten Region des Gens umfasst, der funktionell mit einem Promoter und einem Transkriptionsterminationssignal verbunden ist.

Die Verfahren und einzelne Verfahrensschritte der vorliegenden Erfindung können *in vitro, in vivo* und *ex vivo* ausgeführt werden. In einer bevorzugten Ausführungsform handelt es sich bei den in den Verfahren verwendeten Zellen um nicht-menschliche Zellen. In einer alternativen bevorzugten Ausführungsform handelt es sich bei den verwendeten Zellen um menschliche Zellen, z.B. adulte oder embryonale Stammzellen, hämatopoietische Stammzellen oder Lymphozyten.

Der Begriff "Inaktivierung oder Reduzierung einer endogenen Genfunktion" bedeutet Ausschaltung der Genfunktion oder Reduzierung der Genfunktion um mindestens 50%, vorzugsweise mindestens 75% und stärker bevorzugt mindestens 80% wie z. B. mindestens 90% oder 95% und bis zu 100%. Die Werte der Reduktion werden dabei vorzugsweise verglichen mit einer Zelle, die mit der die RNAᵢ-Selektionskassette enthaltenden Zelle im wesentlichen identisch ist, mit der Ausnahme allerdings, dass sie die RNAᵢ-Selektionskassette nicht enthält. Dies kann z. B. eine Zelle derselben Zelllinie sein oder eine Zelle aus demselben Gewebe mit vorzugsweise demselben Differenzierungsstatus. Erfindungsgemäß wird die endogene Genfunktion durch Expression einer RNAi, die einen kurzen doppelsträngigen und komplementären Abschnitt des endogenen Gens umfasst, inaktiviert oder reduziert. Dadurch wird die Proteinbiosynthese des durch das endogene Gen kodierten Proteins deutlich reduziert oder vollständig verhindert.

Der Begriff "selektionierbare Zelle" bedeutet, dass die Zelle gegenüber anderen Zellen, die vorzugsweise in derselben Zellpopulation auftreten, Eigenschaften aufweist, die gegenüber diesen anderen Zellen einen Überlebensvorteil oder einen Überlebesnachteil darstellen. Auf der Basis dieses Vor- oder Nachteils wird die Zelle bzw. werden die Zellen mit denselben selektionierbaren Eigenschaften gegenüber den anderen Zellen ohne die selektionierbaren Eigenschaften angereichert oder abgereichert werden. Erfindungsgemäß kann die selektionierbare Eigenschaft eine transiente oder stabil verankerte Eigenschaft der Zelle sein. Sie ist erfindungsgemäß vorzugsweise genetisch verankert.

Der Begriff "Einführen [...] in eine eukaryontische Zelle" umfasst alle Techniken die dem Fachmann zum Einbringen von Nukleinsäure in eukaryontische Zellen bekannt sind. Solche Techniken sind beispielsweise Mikroinjektion, Transfektion einschließlich Elektroporation, Lipofektion oder Kalziumphosphatpräzipitation oder jede weitere Aufnahme eines Vektors, der die einzubringende Nukleinsäure enthält. Ein Vektor kann beispielsweise ein viraler Vektor wie z.B. ein Adenoviraler Vektor oder ein lentiviraler Vektor sein Denkbar ist aber auch, dass ein einzelnes Proteinmolekül die Aufnahme in die Zelle vermittelt. Darüber hinaus sind dem Fachmann auch Transfektionstechniken bekannt, die die Verwendung makromolekularer Polymere, z.B. Fullerene einschliessen. Schliesslich bezieht sich der Begriff "Einbringen" auch auf die dem Fachmann bekannten ballistischen Methoden die bei der Transfektion von allen eukaryontischen Zellen, insbesondere aber bei der Transformation von pflanzlichen Zellen verwendet werden.

Die in die Zelle eingebrachte RNAi-Selektionskassette kann als RNA oder DNA in die Zelle eingebracht werden. Entsprechend wird erfindungsgemäß unter dem Vektor ein DNA oder RNA basierter Vektor verstanden. Bevorzugte Vektoren sind dadurch charakterisiert, dass sie Polymerase III abhängige Promotoren enthalten wie beispielsweise H1 oder U6 Promotoren oder anderen Polymerase III abhängigen Promotoren z.B. 5S rRNA, snRNA und tRNA Promotoren (Vergl. Mittal, V. 2004: Improving the effiency of RNA interference. Nature Reviews Genetics, 5, 355-365). Weiterhin bevorzugt sind Vektoren die induzierbare Regulationssequenzen enthalten. Hierzu zählen beispielsweise Tetrazyklininduzierbare oder Ecdysone-induzierbare Regulationssequenzen.

"Expression einer von [...] den mehreren Vektoren kodierten siRNA und vorzugsweise shRNA" wird so verstanden, dass die RNAi Selektionskassette, die erfindungsgemäß auf mehreren Vektoren verteilt sein kann, zur Transkription einer siRNA oder shRNA führt. In den Fällen, in denen die RNAi Selektionskassette auf zumindest zwei Vektoren verteilt ist und die Teile der Kassette von Rekombinaseerkennungssequenzen flankiert werden, ist vor Expression der siRNA oder shRNA eine Rekombination der getrennten Nukleotidsequenzen notwendig.

Die "RNAi-Selektionskassette" umfasst neben einer Promotorsequenz und einer Terminatorsequenz eine Nukleotidsequenz mit bevorzugt 100%iger Komplementarität in der Nukleinsäuresequenz zu dem endogenen selektierbaren Gen. In bestimmten bevorzugten Ausführungsformen ist eine geringere Komplementarität bevorzugt. Hierdurch kann beispielsweise sichergestellt werden, dass ein geringes Maß an Basalexpression des Zielgenes weiterhin vorhanden ist. In einer bevorzugten Ausführungsform wird der transkribierte Teil der RNAi-Selektionskassette in einer Anordnung von Sense und Antisense transkribiert, die durch einen nicht-komplementären Bereich unterbrochen ist. Diese Anordnung wird als "small hairpin (sh) RNA" bezeichnet und enthält die Sequenzen der Ziel-mRNA als imperfektes Palindrom, wobei die beiden Hälften der Anordnung von Sense und Antisense durch eine kurze nicht-palindrome Sequenz getrennt werden. Die nichtpanlindromische Sequenz ist bevorzugt 3 bis 10, 10 bis 100 oder 100 bis 1000 Nukleotide lang. Besonders bevorzugt sind kurze nicht-palindromische Zwischensequenzen von 6 bis 9 Nukleotide Länge, wobei bevorzugt die beiden Nukleotide am 5' Ende der nicht-palindromen Sequenz T's sind. Auch denkbar ist, dass der nicht-palindromische Bereich eine oder mehrere Erkennungssequenzen von Rekombinasen enthält. Beispielsweise könnte die 34 Nukleotide lange loxP site oder die 48 Nukleotide lange frt site im nicht-palindromischen Bereich angeordnet sein.

In einer anderen bevorzugten Ausführungsform sind die interferierenden siRNAs von längeren Vorläufermolekülen abgeleitet, die durch die nachstehend in dieser Beschreibung genannten endogen auftretenden Enzyme ("dicer") zu siRNAs abgebaut werden.

Somit bedient sich das erfindungsgemäße Verfahren eines Phänomens, das im Stand der Technik als RNA-Interferenz (RNAi) bekannt ist. RNAi ist ein endogener zellulärer Regulationsmechanismus, der in Eukaryonten die spezifische postranskriptionelle Inaktivierung der Expression eines Zielgenes bewirkt. Verantwortlich für diese Aktivität sind kurze, etwa 19 bis 29nt lange, doppelsträngige, sogenannte small interfering (si) RNAs (Längenangabe laut Mittal, V. 2004: Improving the effiency of RNA interference. Nature Reviews Genetics, 5, 355-365), die in einen als RISC (RNA induced silencing complex) bezeichneten Effektorkomplex inkorporiert werden, der dann verschiedene Funktionen wie die Degradation der Ziel-mRNA oder eine translationale Suppression derselben vermittelt. RISCs sind Ribonukleotid-komplexe, die verschiedene Proteine, siRNA sowie die komplementäre mRNA enthalten, wobei bisher unklar ist, wie die als slicer bezeichnete Degradationsaktivität vermittelt wird. Ein einzelnes siRNA-Molekül kann im RISC-Komplex nacheinander die Degradation mehrerer mRNA-Moleküle vermitteln.

*In vivo* werden siRNA-Moleküle aus längeren doppelsträngigen Vorläufer-RNAs mittels einer als dicer bezeichneten RNAseIII erzeugt. Entsprechend werden transfizierte längere für eine bestimmte Ziel-mRNA spezifische doppelsträngige RNAs zu biologisch wirksamen siRNAs abgebaut. Eine andere Möglichkeit genspezifische siRNA-Moleküle zu erzeugen besteht in der Transfektion von Polymerase III abhängigen Expressionsvektoren, die sogenannte small hairpin (sh) RNAs exprimieren. Diese enthalten einen 19nt langen Teil der Sequenz der Ziel-mRNA als imperfektes Palindrom, wobei die beiden Hälften des "repeats" durch eine kurze nicht-palindrome Sequenz getrennt werden. Am 3'-Ende des zu exprimierenden Konstruktes befinden sich erfindungsgemäß vorzugsweise 5 konsekutive Thymidine, die von der Polymerase III als transkriptionelles Terminationssignal erkannt werden. In Übereinstimmung mit den vorgenannten Ausführungen wird in einer bevorzugten Ausführungsform der Erfindung in Schritt (a) ein pol III Promotor als Promotor verwendet.

*In vivo* formen entsprechende Transkripte RNA-Doppelhelices mit einem RNA-loop am einen Ende und einem zwei T langen 3'-Überhang am anderen Ende. Durch Wirkung von dicer wird der loop entfernt, so daß ein funktionelles siRNA-Molekül entsteht, das die RISC-vermittelte Inaktivierung der Ziel-mRNA vermitteln kann.

In Säugerzellen durchgeführte Experimente deuten darauf hin, daß es zumindest zwei verschiedene Antworten auf dsRNA gibt, eine unspezifische sowie eine spezifische dsRNA-Antwort, die um die dsRNA kompetitieren. Unspezifische RNAi-Effekte werden unter anderem auf das Vorhandensein eines in Säugerzellen verbreiteten antiviralen Mechanismus zurückgeführt, der auch als Interferonantwort bezeichnet wird. Induktor der unspezifischen dsRNA-Antwort sind längere dsRNA Moleküle, so fern diese zumindest 30 Basenpaare lang sind. Dabei erkennen zelluläre Proteine die dsRNA und initiieren eine allgemeine Inhibition der zellulären Translation (Terenzi et al., 1999; Williams, 1999). Dies führt zu einer unspezifischen Reduktion von Genexpression. Die dsRNA aktiviert hierbei u.a. zwei Enzyme: PKR, welches in seiner aktiven Form den Translationsinitiationsfaktor eIF2a phosphoryliert, was zu einem Abschalten der Proteinsynthese führt, und 2', 5'-Oligoadenylatsynthetase, welche ein Molekül bildet, das RNase L aktiviert, ein nichtspezifisches Enzym, das mRNAs abbaut (Elbashir et al, 2001).

Da mit zunehmender Länge der "small hairpin (sh) RNAs" die Wahrscheinlichkeit einer zellulären Interferonantwort steigt ist bevorzugt, dass die von der RNAi-Selektionskassette transkribierte "small hairpin (sh) RNA" eine Länge von bis zu 200 Nukleotide hat. Noch stärker bevorzugt sind jedoch Längen von bis zu 50 Nukleotide wie bis zu 30 Nukleotide und am stärksten bevorzugt sind "small hairpin (sh) RNA"-Längen von 19 Nukleotiden, 20 Nukleotiden, 21 Nukleotiden, 22 Nukleotiden, 23 Nukleotiden, 24 Nukleotiden, 25 Nukleotiden oder bis zu 29 Nukleotiden. Vorzugsweise hat der zum endogenen Gen komplementäre Abschnitt der RNA eine Länge von 19 Nukleotiden, 20 Nukleotiden, 21 Nukleotiden, 22 Nukleotiden, 23 Nukleotiden, 24 Nukleotiden oder 25 Nukleotiden.

Der Begriff "zumindest 19 Nukleotide langen Abschnitt" bedeutet, dass die RNAi-Selektionskassette eine siRNA bzw. eine shRNA transkribiert, die einen Abschnitt mit einer Länge von zumindest 19 Nukleotiden enthält, wobei dieser Abschnitt komplementär zu einem transkribierten Abschnitt des endogenen zellulären Gens ist. Die siRNA bzw. shRNA kann insgesamt bis 200 Nukleotide lang sein. Dabei liegt aber die Anzahl der mit der Zielsequenz über einen konsekutiven Abschnitt identischen bzw. im wesentlichen identischen Nukleotiden (komplementärer Bereich) vorzugsweise 19 bis zu 50 Nukleotiden, und stärker bevorzugt bei 19, 20, 21, 22, 23, 24 oder 25 Nukleotiden. Vorzugsweise hat der mit dem endogenen Gen komplementäre Abschnitt der siRNA/shRNA" also eine Länge von 19 bis zu 50 Nukleotiden und am stärksten bevorzugt sind Längen von 19 Nukleotiden, 20 Nukleotide, 21 Nukleotide, 22 Nukleotiden, 23 Nukleotiden, 24 Nukleotiden oder 25 Nukleotiden, wobei insbesondere bei shRNAs weitere, den nicht-palindromischen Bereich bildende Nukleotide vorhanden sein können. Definitionsgemäß liegt der nicht-palindromische Bereich zwischen dem antiparallelen Abschnitt, der aus einem Nukleotidabschnitt in "Sense-" und einem Nukleotidabschnitt in "Antisense-Orientierung" gebildet wird. Der Begriff "im wesentlichen identisch" bezeichnet Abschnitte mit unvollständiger Komplementarität. In bestimmten bevorzugten Ausführungsformen ist eine geringere Komplementarität bevorzugt. Hierdurch kann beispielsweise sichergestellt werden, dass ein geringes Maß an Basalexpression weiterhin vorhanden ist. Vorzugsweise haben die im wesentlichen identischen Abschnitte innerhalb der RNAi bzw. shRNA eine Sequenzidentität mit der Zielsequenz von mindestens 90%, mindestens 91%, mindestens 92%, mindestens 93%, mindestens 94%, mindestens 95%, mindestens 96%, mindestens 97%, mindestens 98% oder mindestens 99%. Am stärksten bevorzugt sind im wesentlichen identische Abschnitte von 100% Sequenzidentität mit der Zielsequenz.

Der Begriff "funktionell mit einem Promotor und einem Transkriptionsterminationssignal verbunden" bedeutet, dass eine Transkription der oben näher beschriebene Anordnung aus Sense und Antisense in RNA möglich ist, die durch den Promotor vermittelt und durch das Transkriptionsterminationssignal terminiert wird. Hierbei sind bevorzugte Promotoren, wie vorstehend erwähnt, pol III Promotoren (Review: Paule MR, White RJ (2000) Survey and summary: transcription by RNA polymerases I and III. Nucleic Acids Res. 28(6):1283-1298) z.B. H1 Promotor (Brummelkamp TR, Bernards R, Agami R. (2002): A system for stable expression of short interfering RNAs in mammalian cells. Science 296: 550-553), U6 Promotor (Sui G, Soohoo C, Affar el B, Gay F, Shi Y, Forrester WC, Shi Y (2002): A DNA vector-based RNAi technology to suppress gene expression in mammalian cells. Proc Natl Acad Sci U S A. 99(8):5515-5520; Paul CP, Good PD, Winer I, Engelke DR. (2002): Effective expression of small interfering RNA in human cells. Nat Biotechnol. 20(5):505-508) 5S rRNA und tRNA Promotoren aber auch weniger bevorzgt Polymerase II Promotoren wie z.B. der CMV Promotor (Xia H, Mao Q, Paulson HL, Davidson BL (2002): siRNA-mediated gene silencing in vitro and in vivo. Nat Biotechnol. 20(10):1006-1010; Shinagawa T, Ishii S(2003): Generation of Ski-knockdown mice by expressing a long double-strand RNA from an RNA polymerase II promoter. Genes Dev. 17(11):1340-1345). Bevorzugte Terminationssequenzen für Polymerase III abhängige Promotoren sind mindestens 4 bevorzugt 5 konsekutive Thymidine (T) stromabwärts vom zu transkribierenden Teil des RNAi-Konstruktes (Review: Paule MR, White RJ (2000) Survey and summary: transcription by RNA polymerases I and III. Nucleic Acids Res. 28(6):1283-1298). Auch ist eine Verlängerung um weitere konsekutive T denkbar und von der vorliegenden Erfindung umfasst.

Die dargestellten Ergebnisse zeigen, da das erfindungsgemäße Verfahren, das in einer bevorzugten Ausführungsform HPRT-RNAi-Konstrukte verwendet (s.u.) geeignet ist, die Expression des Zielgens in Zellen soweit herabzusetzen, dass die Zellen ohne unzumutbaren Aufwand selektioniert werden können. Im Falle der genannten bevorzugten Ausführungsform entwickeln die Zellen eine Resistenz gegen 6-Thioguanosin und 8-Azaguanosin und werden gleichzeitig sensitiv für HAT-Medium. Die beobachteten Eigenschaften der transfizierten Zellen stellen also eine proteinfrei erzeugte Phänokopie von Zellen dar, die eine reduzierte/inakvivierte Expression des Zielgens aufweisen.

Nachfolgend werden Vorteile der Erfindung anhand der bevorzugten Ausführungsform HPRT-RNAi-Selektionskassette genauer beschrieben. Erfindungsgemäß kann das Verfahren jedoch auch auf jegliche eukaryontischen selektionierbaren Genfunktionen erstreckt werden, ohne dass es dazu eines unzumutbaren Aufwandes bedürfte. Gemäß der erfindungsgemäßen Lehre und auf der Basis seines allgemeinen Fachwissens kann sich der Fachmann ein geeignetes Gen/Protein auswählen, eine Nukleotidsequenz bestimmen, gegen die die siRNA/shRNA gerichtet ist und das Verfahren als Selektionsverfahren umsetzen.

Weitere bevorzugte Zielgene/Zielproteine werden nachstehend genauer beschrieben.

Ein weiterer Vorteil des erfindungsgemäßen Systems ist, dass der Phänotyp, beispielsweise RNAi-vermittelte HPRT-Defizienz von Zellen, durch die Entfernung der RNAi-Selektionskassette, z.B. mittels Rekombinase-vermittelter Deletion oder durch homologe Rekombination, revertiert werden kann. Die Zellen werden dadurch im Falle unseres Beispiels also wieder HAT-resistent und sensitiv für 6-Thioguanosin und 8-Azaguanosin. Entsprechend ließen sich die mit HPRT-RNAi-Konstrukten stabil transfizierten Zellen wieder in HAT-sensitive Zellen verwandeln, wenn das betreffende Konstrukt wieder aus dem Genom der Zellen entfernt wird, z.B. durch den Einsatz geeigneter Rekombinase-Systeme.

Gegenüber herkömmlichen Selektionssystemen unterscheiden sich RNAi-basierte Selektionssysteme in vorteilhafter Weise auch dadurch, daß sie ohne Expression von Proteinen auskommen. Somit sind sie als nicht-allergen einzustufen, was bei der Herstellung transgener Pflanzen oder beim Einsatz in Vektoren zur Gentherapie von Vorteil sein dürfte. Weiterhin bieten entsprechende Kassetten den Vorteil, daß sie keine Resistenz bzw. Sensitivität in Prokaryonten vermitteln können, da diesen der RNAi-Mechanismus fehlt. Außerdem können die benötigten shRNAs speziesspezifisch hergestellt werden, so daß ein eventueller horizontaler Gentransfer z.B. von einer apathogenen Spezies auf eine pathogene keine Auswirkung haben sollte.

Da eine Kopie eines (erfindungsgemäß bevorzugten) HPRT-RNAi-Konstruktes für die beobachteten Effekte ausreicht, können entsprechende Kassetten zur Einführung von allen gentechnischen Veränderungen in Zellen benutzt werden, für die bisher andere Selektionskassetten (z.B. neo-, hygro- oder puro-Selektionskassetten) benutzt wurden, und somit auch zur Herstellung von gentechnisch veränderten Tieren oder Pflanzen. Dabei sind RNAi-basierte Selektionskassetten aber wesentlich kleiner (<200bp) als herkömmliche, auf der Expression von Proteinen beruhende Selektionskassetten. Diese Verkürzung liegt insbesondere an der Verwendung von RNAIII-abhängigen Promotoren. So ist beispielsweise der H1 Promoter lediglich 100bp lang, während herkömmliche Selektionskassetten mit auf RNA II Polymerase basierenden Promotoren schon aufgrund der Promotorsequenzen und polyA-Signale oftmals wesentlich länger sind (>1000bp) und durch ihre Länge häufig Probleme beim Klonieren verursachen. Des weiteren kommen auch solche Ziel-mRNAs in Frage für die nur Substanzen zur Selektion gegen das Produkt existieren (z.B. Dihydrofolatreduktase, Tabelle 1).

Da eine Kopie des HPRT-RNAi-Konstruktes zur Vermittlung von Resistenz gegen 6-Thioguanosin und 8-Azaguanosin ausreicht, ist außerdem eine Verwendung von RNAi-basierten Selektionskassetten in herkömmlichen und konditionalen gene-trap-Vektoren denkbar.

Ein Nachteil der RNAi-Technik liegt darin, daß sie in Prokaryonten nicht funktioniert. Trotzdem ist eine Verwendung entsprechender Kassetten bei Herstellung von targeting-Konstrukten mittels homologer Rekombination in Bakterien möglich, wenn sie mit einem zweiten Selektionsmarker (z.B. β-Lactamase) in einer Kassette kombiniert werden. Der prokaryontische Selektionsmarker ließe sich dann über Rekombinase-vermittelte Deletion entfernen, so daß in einem so hergestellten Konstrukt die eukaryontisch wirksame RNAi Kassette erhalten bliebe und z.B. zur Herstellung transgener Tiere benutzt werden könnte.

In einer bevorzugten Ausführungsform der Erfindung umfasst das erfindungsgemäße Verfahren den weiteren Schritt (c) der Expression einer Rekombinase, wobei die RNAi-Selektionskassette nach homologer Rekombination gebildet wird, wobei vor der Rekombination zwischen den Rekombinationsvorstufen, zwischen Promotor und RNAi-Selektionskassette oder innerhalb der RNAi-Selektionskassette eine 5' und eine 3' Rekombinaseerkennungssequenz und zwischen diesen eine trennende Nukleotidsequenz angeordnet ist, wobei die trennende Nukleotidsequenz ein Transkriptionsterminationssignal enthält und wobei durch die Expression der Rekombinase eine homologe Rekombination an den Rekombinaseerkennungssequenzen erfolgt. Aufgabe der trennenden Nukleotidsequenz ist es, zunächst die Transkription einer RNAi oder shRNA zu verhindern. Entsprechend ist auch vorstellbar, dass anstatt eines bekannten Transkriptionstterminationssignals beispielsweise zelluläre Nukleotidsequenzen mit ähnlicher Funktion oder Wirkung enthalten sind.

In dieser bevorzugten Ausführungsform der Erfindung wird eine nicht-funktionelle RNAi Selektionskassette in die Zelle eingebracht. Dieser inaktive Zustand der RNAi Selektionskassette wird durch die Anwesenheit von trennende Nukleotidsequenzen innerhalb der RNAi Selektionskassette sichergestellt, wobei die trennenden Nukleotidsequenzen ein Transkriptionsterminationssignal umfassen. Da die trennenden Nukleotidsequenzen von Rekombinaseerkennungssequenzen flankiert werden, können durch Expression einer zu den Erkennungssequenzen passenden Rekombinase die trennenden Nukleotidsequenzen entfernt und somit eine funktionelle RNAi Selektionskassette gebildet werden. Wie weiter unten ausgeführt wird, können die Rekombinaseerkennungssequenzen beispielsweise zwischen der ersten und zweiten Sequenz der invertierten Sequenzwiederholung der shRNA angeordnet sein. Weiterhin ist es möglich die RNAi Selektionskassette auf mehrere Vektoren aufzuteilen und erst nach einem Rekombinationsereignis zusammen zu bringen, d.h. in einen funktionellen Zustand zu überführen.

Bevorzugt werden die Rekombinaseerkennungssequenzen ausgewält aus der Gruppe bestehend aus loxP, frt, attb-attp sowie mutierte Formen davon. Durch Expression der betreffenden Rekombinase Cre, flp, phiC31 wird der Bereich zwischen den Rekombinaseerkennungssequenzen deletiert, so dass das RNAi-Konstrukt rekonstituiert und aktiviert wird und somit für die betreffende Deletion selektioniert werden kann.

In einer stärker bevorzugten Ausführungsform des erfindungsgemäßen Verfahrens sind die Rekombinaseerkennungssequenzen zwischen der ersten und zweiten Sequenz der invertierten Sequenzwiederholung der shRNA angeordnet. Hierbei bezieht sich der Begriff "invertierte Sequenzwiederholung" auf die weiter oben beschriebene Anordnung aus Nukleotidsequenzen in "sense" und "antisense" Orientierung. Durch Expression der betreffenden Rekombinase wird der Bereich zwischen den Rekombinaseerkennungssequenzen deletiert, so dass das RNAi-Konstrukt rekonstituiert wird, wobei die eine verbleibende Rekombinaseerkennungssequenz den loop, also den nicht-palindromischen Abschnitt im exprimierten shRNA-Molekül bildet. Mit dem so aktivierten RNAi-Konstrukt kann somit für die betreffende Deletion selektioniert werden. Liegen die "invertierten Sequenzwiederholungen" in zwei getrennte Positionen eines Chromosoms, kann durch Expression der betreffenden Rekombinase der von den Hälften des RNAi-Konstruktes flankierte Chromosomenbereich deletiert werden, wobei die Rekonstituierung des RNAi-Konstruktes eine Selektion für die erfolgte Deletion ermöglicht.

In einer anderen stärker bevorzugten Ausführungsform des erfindungsgemäßen Verfahrens sind (i) der Promoter, die 5' Rekombinaseerkennungssequenz und die erste Sequenz der invertierten Sequenzwiederholung der shRNA und (ii) die 3' Rekombinaseerkennungssequenz und die zweite Sequenz der invertierten Sequenzwiederholung der shRNA auf unterschiedlichen Vektoren angeordnet. Bei diesem Verfahren sind die beiden Hälften, d.h. der Promotor mit einer Rekombinaseerkennungssequenz und die zweite Rekombinaseerkennungssequenz mit der bei der RNA-Interferenz wirksamen transkribierten RNAi-Sequenz, in trans, d.h. auf unterschiedlichen Molekülen, bevorzugt unterschiedlichen Vektoren bzw. Chromosomen angeordnet. Die Wirkung der betreffenden Rekombinase nach Expression derselben führt zu intermolekularer Rekombination, bevorzugt interchromosomaler Rekombination, d.h. Translokation der betreffenden Abschnitte der Chromosomen, und zur Rekonstitution eines funktionellen RNAi-Konstruktes, so daß für die intermolekulare Rekombination selektioniert werden kann.

In einer anderen bevorzugten Ausführungsform umfasst das erfindungsgemäßen Verfahren den weiteren Schritt (d) der Kultivierung und Anreicherung von Zellen die die RNAi-Selektionskassette enthalten und exprimieren oder (d') Kultivierung und Anreicherung von Zellen die die RNAi-Selektionskassette nicht enthalten und exprimieren. Sollte das erfindungsgemäße Verfahren beispielsweise den Schritt (c) nicht umfassen sind die hier genannten Schritte (d) und (d') entsprechend als Schritte (c) und (c') zu verstehen. Entsprechendes gilt für die weiteren in der Anmeldung erwähnten Verfahrensschritte und deren Kombinationen. Die Variante (d') Kultivieren und Anreicherung von Zellen die die RNAi-Selektionskassette nicht enthalten und exprimieren als zweiter Verfahrensschritt kann in solchen Fällen angewendet werden, in denen gegen die Expression des RNAi-Konstruktes selektioniert werden soll. Vorteilhafterweise kann dieses System eingesetzt werden, wenn gegen die zufällige Integration eines genomischen Konstruktes selektioniert werden soll. Eine RNAi-Selektionskassette wird so konstruiert, dass die in das Genom einzuführende gewünschte DNA von zu der Zielsequenz homologen Sequenzen flankiert wird. An das eine Ende zumindest einer flankierenden Sequenz wird das eine RNAi-Selektionskassette kloniert, die mit der Expression eines für die Zelle essentiellen Gens interferiert. Dieses essentielle Gen kann beispielsweise ein Strukturprotein wie Aktin oder ein Enzym wie die Polymerase II kodieren. Bei Integration in den gewünschten Bereich geht die RNAi-Selektionskassette verloren, so das es nicht zur Interferenz mit der Expression des essentiellen Gens kommt. Sofern andererseits eine zufällige Integration stattfindet, besteht eine große Chance, dass das RNAi-Konstrukt mit in das Genom integriert wird. In diesem Falle kommt es zur Inhibition oder Reduktion der essentiellen Genfunktion und damit zum Absterben oder zu Wuchsnachteilen der Zelle.

In einer bevorzugten Ausführungsform der Erfindung ist das selektionierbare Gen positiv und/oder negativ selektionierbar. "Positiv selektionierbar" bedeutet erfindungsgemäß das Anreichern von Zellen, die RNAᵢ Expression aufweisen. "Negativ selektionierbar" bedeutet erfindungsgemäß das Anreichern von Zellen, die keine RNAᵢ Expression aufweisen.

In einer anderen bevorzugten Ausführungsform der Erfindung kodiert das endogene selektionierbare Gen ein Produkt mit der Fähigkeit, eine nicht selektierende Vorstufe einer Substanz A in ein Produkt B mit selektionierenden oder selektionierbaren Eigenschaften umzuwandeln und die Zelle wird in Gegenwart der Substanz A kultiviert.

In einer weiteren bevorzugten Ausführungsform der Erfindung ist die nicht selektierende Substanz A eine ungiftige Vorstufe eines Toxins und das Produkt B ist ein Toxin. Der Begriff Toxin bezieht sich auf eine chemische Verbindung die einen hemmenden Einfluss auf das Zellteilung bzw. das Zellwachstum hat. Beispiele für Toxine sind Nukleotidanaloga die zum Kettenabbruch bei der Synthese von DNA-Sequenzen führen. Weitere Beispiele sind in Tabelle 1 genannt. Entsprechend der erfindungsgemäßen technischen Lehre führt die Expression der oben näher beschriebenen RNAi bzw. shRNA dazu, dass Zellen für bestimmte Substanzen, d.h. beispielsweise für die in Tabelle 1 aufgeführten Substanzen sensitiv werden, wobei Sensitivität bedeutet, dass Zellen nicht mehr wachsen oder absterben.

Ähnlich zu verwendende Konstrukte wie das hier beschriebene HPRT-RNAi-Konstrukt sind auch für andere endogene mRNAs verfügbar, für andere RNAs, für bzw. gegen deren Produkte mittels exogen zugegebener Substanzen selektioniert werden kann (z.B. Thymidinkinase und APRT). Eine Übersicht besonders bevorzugter Gene/Genprodukte und diese selektionierende Substanzen ist in Tabelle 1 angeben.

**Tabelle 1:**

| Wirkung verschiedener Selektionsmedien auf Wildtypzellen (Wildtyp), auf Zellen die gegen HPRT, APRT Thymidin Kinase bzw. DHFR gerichtete RNAi-Konstrukte exprimieren (RNAi) und auf Zellen, in denen die entsprechenden Gene auf chromosomaler Ebene inaktiviert vorliegen (kock out). Überleben der Zellen in dem jeweiligen Selektionsmedien wird mit + gekennzeichnet, Absterben der Zellen unter den jeweiligen Selektionsbedingungen mit - gekennzeichnet. | | | | |
|---|---|---|---|---|
| **Gen (HomoloGene- Eintrag; HomoloGene, build 36, last update 05/25/2004)** | **Selektionsmedium** | **Wildtyp** | **RNAi** | **knock out** |
| HPRT (162) | HAT | + | - | - |
| | 8-Azaguanosin oder 6-Thioguanosin | - | + | + |
| APRT (413) | ALASA (Alanosin/Azaserin/Adenine) | + | - | - |
| | Diaminopurine, 8-Azaadenine oder 2-Fluoroadenine | - | + | + |
| Thymidine Kinase (2446) | HAT oder CHAT | + | - | - |
| | Trifluorothymidine oder Bromodesoxyuridine | - | + | + |
| DHFR (619) | Methotrexate | + | - | - |

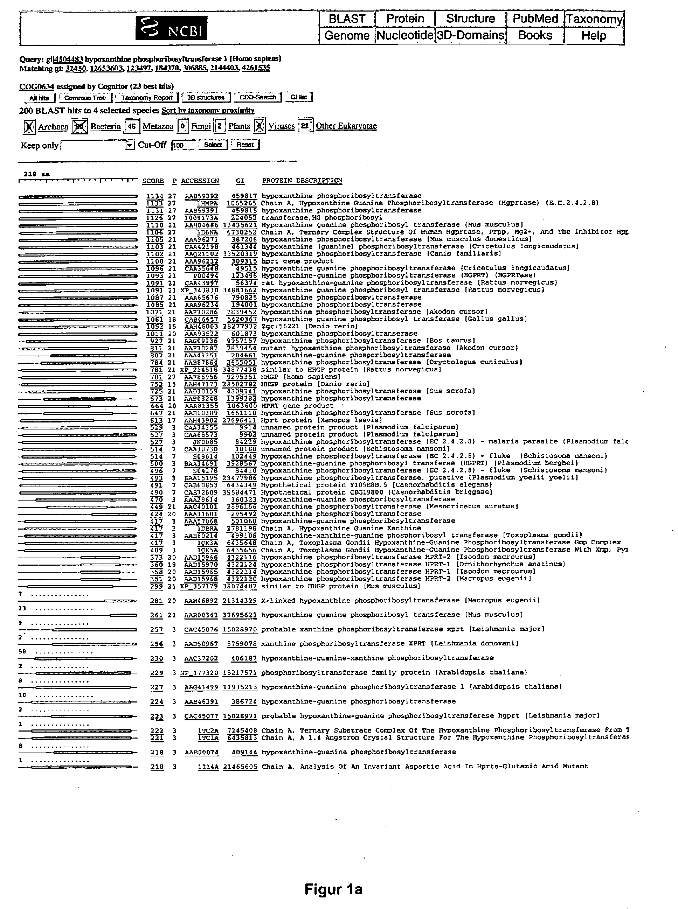

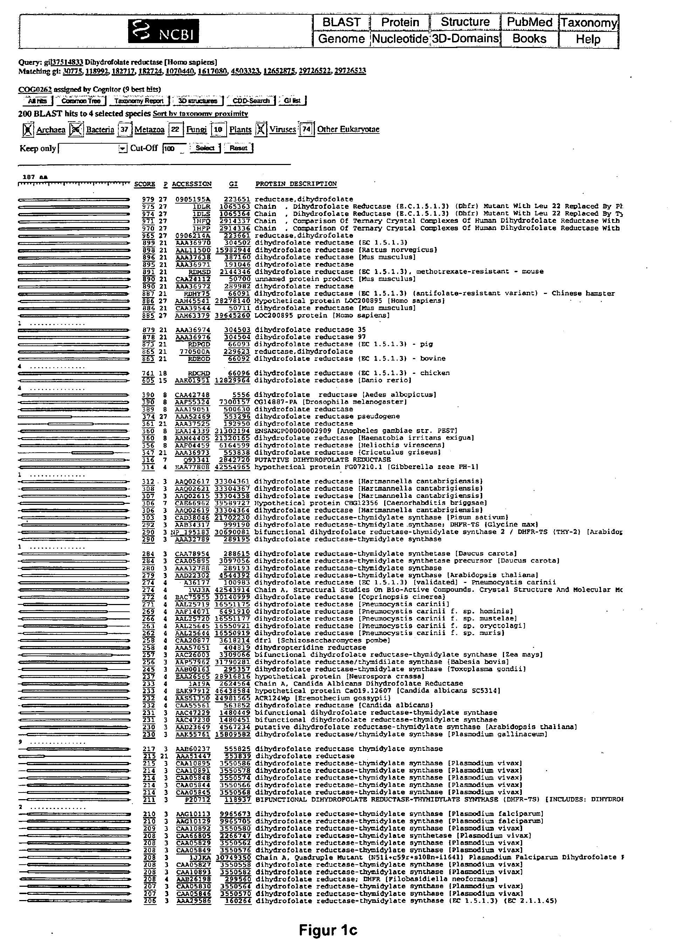

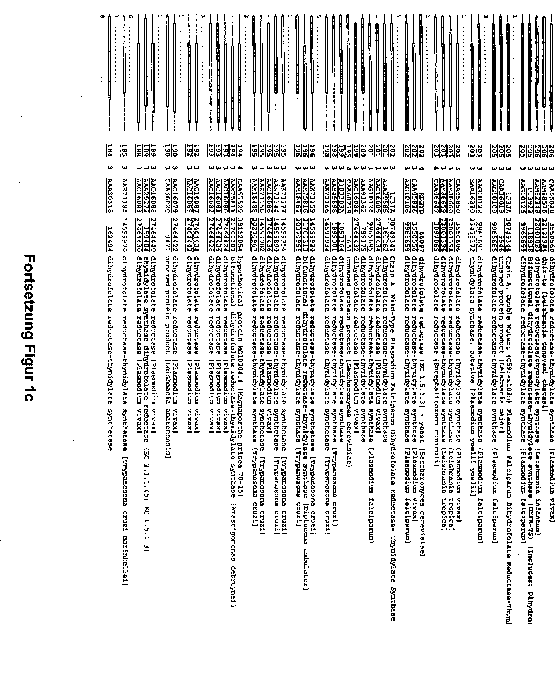

Gemäß vorstehenden Überlegungen ist in einer besonders bevorzugten Ausführungsform der Erfindung das selektionierbare Gen APRT, DHFR oder TK. In einer weiteren besonders bevorzugten Ausführungsform der Erfindung ist, wie oben angedeutet, das selektionierbare Gen HPRT. Die vorgestellt genannten Gene umfassen alle bekannten allelischen Varianten der Gene, in allen Spezies. Besonders bevorzugt sind die in der HomoloGene-Datenbank des NCBI (HomoloGene build 36, last update 05/25/2004) aufgeführten Gene: HPRT HomoloGene: 162, APRT Homologene: 413, Thymidine Kinase HomoloGene: 2446, DHFR HomoloGene: 619), die in Figur 1a-d aufgeführt sind. Die Hinterlegungsnummem der in der Genbank hinterlegten Sequenzen sind den Abbildungen zu entnehmen. Hierbei steht HPRT für die Hypoxanthin-Guanin Phosphoribosyltransferase (HPRT; EC 2.4.2.8), die eine wichtige Rolle im Stoffwechsel der Purinbasen Hypoxanthin und Guanine spielt und insbesondere bei der Fusion von Myelomazellen und B-Zellen in der Herstellung von Hybridomen eine herausragende Rolle spielt (siehe z.B Harlow and Lane "Antibodies, A Laboratory Manual", CSH Press, Cold Spring Harbor, 1998).

Erfindungsgemäß ebenfalls besonders bevorzugt ist eine Ausführungsform, bei der 6-Thioguanosin oder 8-Azaguanosin zur Selektion verwendet werden. In einer weiteren, besonders bevorzugte Ausführungsform der Erfindung werden ALASA (Alanosin/Azaserin/Adenine), Diaminopurine, 8-Azaadenine oder 2-Fluoroadenine, HAT oder CHAT, Trifluorothymidine oder Bromodesoxyuridine oder Methotrexate zur Selektion verwendet.

In einer anderen bevorzugten Ausführungsform der Erfindung wird zusammen mit der RNAi-Selektionskassette oder deren Rekombinationsvorstufe, oder nachfolgend zumindest eine weitere Nukleotidsequenz in die Zelle eingebracht. Die homologen genomischen Nukleotidsequenzen sind vorzugsweise so beschaffen, dass eine homologe Rekombination in die zelluläre genomische DNA möglich ist. Vorzugsweise ist die flankierende, 5' und 3' gelegene homologe genomische Nukleotidsequenz bis zu 100bp, stärker bevorzugt bis zu 1000bp, noch stärker bevorzugt bis zu 10000bp und am stärksten bevorzugt bis zu 200000bp lang. Idealerweise zeigt die flankierende Nukleotidsequenz 100% Sequenzidentität, es ist aber auch denkbar flankierende Sequenzen mit geringerer Sequenzidentität zu verwenden. "Homologe Nukleotidsequenzen" bezeichnen dementsprechend Nukleinsäureabschnitte, die über die gesamte Sequenzlänge Sequenzidentität von mindestens 50% aufweisen, bevorzugt mindestens 70%, stärker bevorzugt mindestens 90%, noch stärker bevorzugt mindestens 95% und insbesondere bevorzugt 100% Sequenzidentität. Die Sequenzidentität wird vorzugsweise durch die FASTA, BLAST (Basic Local Alignment Search Tool) oder Bestfit Algorithmen des GCG-Sequenzanalyseprogramms bestimmt (Wisconsin Sequence Analysis Package, Version 8 for Unix, Genetics Computer Group, University Research Park, 575 Madison, WI 53711). Bei der Verwendung von Bestfit werden die Parameter vorzugsweise so eingestellt, dass der prozentuale Anteil der Identität über die gesamte Länge der Referenzsequenz berechnet wird und Homologielücken ("gaps") von bis zu 5% der Gesamtzahl der Nukleotide erlaubt sind. Bei der Verwendung von Bestfit werden die sogenannten optionalen Parameter vorzugsweise bei ihren voreingestellten Werten belassen.

In einer weiteren bevorzugten Ausführungsform der Erfindung sind die zumindest eine weitere Nukleotidsequenz zwei Nukleotidsequenzen mit Homologie zu zellulären genomischen Nukleotidsequenzen, die die RNAi-Selektionskassette oder deren Rekombinationsvorstufe an deren 5' und 3' Ende flankieren. Die weitere Nukleotidsequenz kann ein Gen oder Genfragment sein. Vorzugsweise ist die weitere Nukleotidsequenz eine endogene oder exogene Nukleotidsequenz. Der Begriff "exogene Nukleotidsequenz" bezieht sich auf eine Nukleotidsequenz, die von aussen eingeführt wurde und keine Entsprechung in der Zelle findet. Beispiele von exogenen Sequenzen sind bakterielle Gene oder Fusionskonstrukte. Entsprechend bezieht sich der Begriff "endogene Nukleotidsequenz" auf eine Nukleotidsequenz die eine Entsprechung in der Zelle findet.

In einer stärker bevorzugten Ausführungsform der Erfindung ist die weitere in die Zelle eingebrachte Nukleotidsequenz auf dem Vektor gelegen, der die RNAᵢ-Selektionskassette enthält und wird die weitere Nukleotidsequenz von den 5' und 3' flankierenden Sequenzen mit Homologie zu zellulären Nukleotidsequenzen umfasst. Flankierende Sequenzen die zur homologen Rekombination benötigt werden sind vorzugsweise bis zu 100bp, stärker bevorzugt bis zu 1000bp, noch stärker bevorzugt bis zu 10000bp und am stärksten bevorzugt bis zu 200000bp lang.

In einer weiteren besonders bevorzugten Ausführungsform der Erfindung ist die weitere in die Zelle eingebrachte Nukleotidsequenz (in allen erfindungsgemäßen Ausführungsformen z.B. ein Gen oder eine exprimierbare kodierende Sequenz, die ein Protein oder Peptid mit einer gewünschten Eigenschaft kodiert) auf einem separaten Vektor gelegen und sind 5' und 3' der weiteren Nukleotidsequenz Nukleotidsequenzen mit Homologie zu zellulären genomischen Nukleotidsequenzen vorhanden.

Besonders bevorzugt ist ferner gemäß einer weiteren Ausführungsform der Erfindung, dass die weitere in die Zelle eingebrachte Nukleotidsequenz nachfolgend in die Zelle eingebracht wird und die weitere Nukleotidsequenz 5' und 3' von homologen genomischen Nukleotidsequenzen flankiert wird, die eine Rekombination der weiteren Nukleotidsequenz in die chromosomale Position der RNAi-Expressionskassette ermöglichen. Vorzugsweise wird durch die Rekombination in die Position der RNAᵢ-Expressionskassette die weitere Expression der RNAᵢ verhindert, d.h. die RNAᵢ-Expressionskassette wird durch homologe Rekombination entfernt. Stärker bevorzugt werden die flankierenden Nukleotidsequenzen so konzipiert, dass die RNAi-Expressionskassette vollständig aus dem Chromosom entfernt wird. Hierfür sind beispielsweise die flankierenden Nukleotidsequenzen der RNAi-Expressionskassette und der nachfolgend eingebrachten Nukleotidsequenz gleich oder sie überlappen.

In einer weiteren besonders bevorzugten Ausführungsform der Erfindung wird die RNAi-Selektionskassette und/oder die weitere in die Zelle eingebrachte Nukleotidsequenz zusätzlich von Rekombinase-Erkennungssequenzen flankiert. Bevorzugt sind die Erkennungssequenzen loxP, frt, attb-attp sowie mutierte Formen von loxP, frt und attb-attp Erkennungssequenzen oder ähnliche für die Rekombinasen cre, flp und phiC31 oder andere Rekombinasen (es werden noch welche dazukommen). Sofern die Konstrukte zu einem Zielgen homologe Strukturen aufweisen, sind die homologen Strukturen vorzugsweise endständig von den Rekombinase-Erkennungssequenzen lokalisiert, so dass letztere bei einem Rekombinationsereignis im in das Genom integrierten Konstrukt erhalten bleiben.

Besonders bevorzugt ist erfindungsgemäß ebenfalls ein Verfahren, in dem die weitere in die Zelle eingebrachte Nukleotidsequenz ein Gen oder ein Fragment davon ist.

Besonders bevorzugt ist gemäß einer anderen Ausführungsform des erfindungsgemäßen Verfahrens, dass das Gen eine Mutante oder allelische Variante eines endogenen Gens der Zelle ist. Der Begriff allelische Variante bedeutet, daß sich die allelische Variante in mindestens einem Nukleotid von dem betreffenden Gen unterscheidet. Darüber hinaus umfasst das erfindungsgemäße Verfahren auch Ausführungsformen, in denen ein orthologes Gen in die Zelle eingeführt wird. Falls die Zelle eine humane Zelle ist, sind besonders bevorzugte Ausführungsformen solche, in denen das Gen von Maus stammt. Falls die Zelle eine murine Zelle ist, sind besonders bevorzugte Ausführungsformen solche, in denen das Gen vom Menschen stammt. Das Gen kann sowohl auf demselben Chromosom und an die Position des entsprechenden zellulären Gens inseriert werden, oder an eine andere Position, beispielsweise innerhalb des selben Chromosoms oder auf einem anderen Chromosom.

In einer weiteren besonders bevorzugten Ausführungsform der Erfindung ist das Gen ein exogenes Gen. Der Begriff "exogenes Gen" umfasst Gene die keine Entsprechung in der Zelle finden. Hierzu zählen beispielsweise bakterielle Gene oder Fusionkonstrukte die in die Zelle eingefügt werden sollen.

In einer anderen bevorzugten Ausführungsform der Erfindung umfasst das erfindungsgemäße Verfahren ferner die folgenden Schritte: (e) Inaktivierung oder Reduktion der RNAi-Expression; und (f) Selektion und Anreicherung von Zellen, die keine RNAi-basierte Inaktivierung oder Reduktion des endogenen selektionierbaren Gens aufweisen. Inaktivierung kann beispielsweise durch Rekombination in die Position der RNAᵢ-Selektionskassette erfolgen. Auch könnte die RNAi-Selektionskassette durch einen konditional wirksamen Promotor gesteuert werden oder durch entsprechende, dem Fachmann bekannte Regulationselemente reguliert sein. Denkbar ist beispielsweise eine Regulation durch Tetracyclin- oder Ecdysonregulierbare Kontrollelemente

Im Prinzip kann diese bevorzugte Ausführungsform des erfindungsgemäßen Verfahrens auf alle Gene/Genprodukte angewandt werden, die nach Inaktivierung oder signifikanter Reduktion der RNAi selektioniert werden können. Hierfür ist wiederum der Einsatz des HPRT-Gens als Ziel der Interferenz ein hervorragendes Beispiel. Insbesondere kann die HPRT-basierte Selektion auch zur Herstellung alleler Serien mit unterschiedlichen Mutationen des gleichen Allels benutzt werden. Dazu wird das betreffende Allel zunächst durch homologe Rekombination mit dem HPRT-RNAi-Konstrukt versehen und mit 6-Thioguanosin bzw. 8-Azaguanosin selektioniert. Anschließend werden dann genomische Konstrukte, welche die gewünschten Mutationen aber keinen Selektionsmarker enthalten in die entsprechenden, 6-Thioguanosin bzw. 8-Azaguanosin selektionierten Klone transfiziert. Mittels HAT-Selektion können dann homolog rekombinierte Klone mit den gewünschten Mutationen selektioniert. Prinzipiell können die mit 6-Thioguanosin bzw. 8-Azaguanosin selektionierten Klone expandiert werden und Zellen davon parallel mit den gewünschten genomischen Konstruktion transfiziert und mit HAT selektioniert werden. So können beispielsweise allele Varianten eines Gens hergestellt werden, die sich jeweils nur in einem einzigen Nukleotid oder einem einzelnen Codon voneinander unterscheiden. Selbstverständlich können auch solche Varianten hergestellt werden, die sich in mehr als einem Nukleotid oder Codon unterscheiden. Weniger bevorzugt, aber ebenfalls vom Schutzumfang der Erfindung umfasst sind Ausführungsformen, in denen allele Varianten durch serielle und altemierende Transfektion mit einer HPRT-RNAi-Selektionskassette und mit genomischen Konstruktion hergestellt werden.

In einer besonders bevorzugten Ausführungsform der Erfindung ist die Inaktivierung der RNAi Expression eine Rekombinase-vermittelte Deletion und umfasst den Schritt der Expression einer Rekombinase. Hierfür ist die RNAi-Seletionskassette beispielsweise 5' und 3' von Erkennungssequenzen einer Rekombinase flankiert.

Im Zusammenhang mit der hier diskutierten bevorzugten Ausführungsform des erfindungsgemäßen Verfahrens sind die beschriebenen RNAi-Selektionskassetten und bevorzugt eine HPRT-RNAiSelektionskassette beim sogenannten recombinase mediated cassette exchange (RMCE) vorteilhaft (siehe EP-A1-0939120 und Bode J, Schlake T, Iber M, Schubeler D, Seibler J, Snezhkov E, Nikolaev L. (2000): The transgeneticist's toolbox: novel methods for the targeted modification of eukaryotic genomes. Biol Chem. 381(9-10):801-813), bei dem eine von zwei unterschiedlichen Rekombinase-Erkennungssequenzen (z. B. einer Wildtyp- und einer mutierten Erkenungssequenz einer Rekombinase(cre, flp, phiC31 oder andere) flankierte RNAiSelektionskassette zunächst homolog in einen Locus rekombiniert wird und dann mittels der betreffenden Rekombinase gegen eine beliebige Sequenz ausgetauscht werden kann, die von den gleichen Erkennungssequenzen flankiert ist. Dies bietet den Vorteil, dass mit der gleichen Kassette zunächst für die Integration derselben und anschließend für das RMCE-Ereignis selektioniert werden kann. Außerdem dürfte die geringe Größe der Kassette von Vorteil sein, da die Effizienz von Rekombinasen abhängig von der Entfernung ihrer Erkennungssequenzen ist.

In einer weiteren besonders bevorzugten erfindungsgemäßen Ausführungsform und wie vorstehend erwähnt ist das endogene selektionierbare Gen HPRT und erfolgt die Selektion von Zellen, die keine RNAi-basierte Inaktivierung des endogenen selektionierbaren Gens aufweisen in Gegenwart von HAT-Medium (siehe Tabelle 1).

Erfindungsgemäß betrifft eine weitere bevorzugte Ausführungsform ein Verfahren, bei dem der Vektor eine weitere Kassette enthält, die bei zufälliger Integration in ein zelluläres Gen zur Inaktivierung desselben führt und somit als gene trap dient (Stanford WL, Cohn JB, Cordes SP (2001): Gene-trap mutagenesis: past, present and beyond. Nat Rev Genet 2(10):756-768.

Die Anwendung des beschriebenen Verfahrens bleibt nicht auf Zellen von Säugetieren beschränkt, sondern kann in Zellen aus allen Organismen angewendet werden, in denen RNAi möglich ist. Somit ist das System bei der Mehrzahl der Eukaryonten anwendbar. Z.B. könnten APRT-RNAi-Konstrukte in Pflanzen Verwendung finden. Der RNAi-Ansatz bietet dabei wie in Säuger-Zellen den Vorteil, dass Wildtyp-Zellen benutzt werden können, während bisher verfügbare APRT-Selektionssysteme auf Pflanzenzellen ohne ein funktionelles APT1-Gen angewiesen sind, die nur von wenigen Spezies verfügbar sind. Da wie bei dem oben beschriebenen HPRT-RNAi-System für APRT ebenfalls positiv und negativ wirkende Selektionsagentien bekannt sind, böte dies, natürlich unter dem Vorbehalt der Einsetzbarkeit des Systems im Freiland, den Vorteil, dass bei der Saatgutproduktion auch für den Wildtyp selektioniert werden, das Saatgut ggfs. also sortenrein, ohne Kontamination mit Transgenen hergestellt werden könnte.

Entsprechend den vorstehenden Darlegungen ist in einer anderen bevorzugten Ausführungsform der Erfindung die eukaryontische Zelle eine Pflanzenzelle. Der Transfer des Vektors erfolgt nach dem Fachmann bekannten Methoden, vorzugsweise unter Verwendung von Plasmiden, insbesondere solchen Plasmiden, die eine stabile Integration des DNA-Moleküls in das Genom transformierter Pflanzenzellen gewährleisten, beispielsweise binären Plasmiden oder Ti-Plasmiden des Agrobacterium tumefaciens-Systems. Neben dem Agrobacterium-System kommen andere Systeme zur Einführung von DNA-Molekülen in pflanzliche Zellen in Frage, wie z.B. das sogenannte biolistische Verfahren oder aber die Transformation von Protoplasten (vgl. Willmitzer L. (1993), Transgenic Plants, Biotechnology 2; 627-659 für eine Übersicht). Verfahren zur Transformation monokotyler und dikotyler Pflanzen sind in der Literatur beschrieben und sind dem Fachmann bekannt.

Um die Expression von RNAi-Expressionskassetten in pflanzlichen Zellen zu gewährleisten, können diese im Prinzip unter die Kontrolle eines beliebigen in pflanzlichen Zellen funktionalen Promotors gestellt werden. Die Expression der besagten RNAi-Selektionskassette und der weiteren Nukleotidsequenz kann generell in jedem Gewebe einer aus einer transformierten Pflanzenzelle regenerierten Pflanze und zu jedem Zeitpunkt stattfinden, bevorzugt jedoch findet sie in solchen Geweben statt, in denen eine veränderte Fähigkeit zur Bildung und Verwertung von bestimmten Protein von Vorteil entweder für das Wachstum der Pflanze oder für die Bildung von Inhaltsstoffen innerhalb der Pflanze ist. Hierbei wird das besagte bestimmte Protein von der in die Zelle eingebrachten weiteren Nukleotidsequenz kodiert.

Das erfindungsgemäße Verfahren kann zur Herstellung von transgenen Pflanzen verwendet werden, die bestimmte Proteine exprimieren. Hierbei kann es sich um die Überexpression eines endogenen Proteins oder die Expression eines fremden Proteins handeln. Diese sogenannten fremden Proteine könnten beispielsweise von bestimmten Allelen eines Gens kodiert werden. Die technische Lehre der Erfindung umfasst sowohl die zusätzliche Expression eines Allels, wie auch "knock-out " Verfahren.

Bei den erfindungsgemäßen transgenen Pflanzenzellen kann es sich grundsätzlich um Zellen jeder beliebigen Pflanzenspezies handeln. Von Interesse sind sowohl Zellen monokotyler als auch dikotyler Pflanzenspezies, insbesondere Zellen stärkespeichernder oder landwirtschaftlicher Nutzpflanzen, wie z.B. Roggen, Hafer, Gerste, Weizen, Kartoffel, Mais, Reis, Erbse, Zuckerrübe, Tabak, Baumwolle, Wein, Tomate usw. oder Zellen von Zierpflanzen.

In einer stärker bevorzugten Ausführungsform der Erfindung ist die Pflanzenzelle ein Protoplast.

In einer weiteren bevorzugten Ausführungsform der Erfindung ist die eukaryontische Zelle eine Vertebratenzelle oder Invertebratenzelle. Besonders bevorzugte Invertebraten werden ausgewählt aus der Gruppe bestehend aus C. elegans und D. melanogaster. Besonders bevorzugte Vertebraten werden ausgewählt aus der Gruppe bestehend aus Zebrafisch, Mensch, Maus, Ratte, Schwein, Rind und Primat.

In einer stärker bevorzugten Ausführungsform der Erfindung ist die Vertebratenzelle eine Säugerzelle.

In einer besonders bevorzugten Ausführungsform der Erfindung ist die Säugerzelle eine Zelle die ausgewählt wird aus Mensch, Maus, Ratte, Schwein, Rind und Primat.

In einer weiteren besonders bevorzugten Ausführungsform der Erfindung ist die Zelle eine embryonale Stammzelle, adulte Stammzelle, hämatopoetische Stammzelle, Körperzelle, oder eine Zelle einer etablierten Zelllinie.

Weiterhin betrifft die Erfindung eine eukaryontische Zelle, umfassend eine RNAi-Selektionskassette die gegen ein endogenes selektionierbares Gen gerichtet ist, und dessen Funktion inaktiviert oder reduziert. wobei die RNAi-Selektionskassette einen zumindest 19 Nukleotide langen Abschnitt des Gens umfasst, der funktionell mit einem Promotor und einem Transkriptionsterminationssignal verbunden ist.

In einer bevorzugten Ausführungsform der Erfindung wird das endogene selektionierbare Gen ausgewählt aus der Gruppe bestehend aus HPRT, APRT, DHFR oder TK. Die vorgestellt genannten Gene umfassen alle bekannten allelischen Varianten in allen Spezies. Besonders bevorzugt sind die in der HomoloGene-Datenbank des NCBI (HomoloGene build 36, last update 05/25/2004) aufgeführten Gene: HPRT HomoloGene: 162, APRT Homologene: 413, Thymidine Kinase HomoloGene: 2446, DHFR HomoloGene: 619) (siehe Figur 1a-d).

In einer weiteren bevorzugten Ausführungsform der Erfindung ist die RNAi-Selektionskassette stabil in das Genom der Zelle integriert.

Eine andere bevorzugte Ausführungsform der Erfindung betrifft eine Zelle, die eine Pflanzenzelle ist. Bei den erfindungsgemäßen transgenen Pflanzenzellen kann es sich grundsätzlich um Zellen jeder beliebigen Pflanzenspezies handeln. Von Interesse sind sowohl Zellen monokotyler als auch dikotyler Pflanzenspezies, insbesondere Zellen stärkespeichernder oder landwirtschaftlicher Nutzpflanzen, wie z.B. Roggen, Hafer, Gerste, Weizen, Kartoffel, Mais, Reis, Erbse, Zuckerrübe, Tabak, Baumwolle, Wein, Tomate usw. oder Zellen von Zierpflanzen.

In einer besonders bevorzugten Ausführungsform der Erfindung ist die Pflanzenzelle ein Protoplast.

Die Erfindung betrifft weiterhin eine transgene Pflanze, die die erfindungsgemäße Pflanzenzelle enthält. Derartige Pflanzen können beispielsweise hergestellt werden durch Regeneration aus erfindungsgemässen Pflanzenzellen nach dem Fachmann bekannten Methoden. Gegenstand der Erfindung ist ferner Vermehrungsmaterial erfindungsgemässer Pflanzen, das erfindungsgemässe Zellen enthält. Hierzu zählen beispielsweise Stecklinge, Samen Früchte, Wurzelstöcke, Knollen, Sämlinge etc.

In einer anderen bevorzugten Ausführungsform der Erfindung ist die Zelle eine Vertebratenzelle oder Invertebratenzelle. Besonders bevorzugte Invertebraten werden ausgewählt aus der Gruppe bestehend aus C. elegans, D. melanogaster. Besonders bevorzugte Vertebraten werden ausgewählt aus der Gruppe bestehend aus Zebrafisch, Maus, Mensch.

In einer stärker bevorzugten Ausführungsform der Erfindung ist die Vertebratenzelle eine Säugerzelle.

Besonders bevorzugt ist erfindungsgemäß eine Ausführungsform, wobei die Säugerzelle eine Zelle ist, die ausgewählt wird aus der Gruppe bestehend aus Mensch, Maus, Ratte, Schwein, Rind und Primat.

In einer weiteren besonders bevorzugten erfindungsgemäßen Ausführungsform ist die Zelle eine embryonale Stammzelle, eine adulte Stammzelle, eine hematopoetische Stammzelle, Körperzelle oder eine Zelle einer etablierten Zellinie.

Die Erfindung betrifft weiterhin ein Verfahren zur Herstellung eines transgenen Tieres, wobei (a) Zellen nach einem oder mehreren der erfindungsgemäßen Verfahrensschritte behandelt werden und (b) aus den Zellen ein lebensfähiger Organismus gezogen wird. Dieses Verfahren ist besonders geeignet für die Herstellung transgener Vertebraten und Invertebraten.

Die im erfindungsgemäßen Verfahren eingesetzten RNAi-basierten Selektionskassetten ließen sich insbesondere in vorteilhafter Weise ferner zur Herstellung von transgenen Mäusen, vorzugsweise durch ES-Zell-Transfektion sowie für gentechnische Veränderungen mittels homologer Rekombination in embryonalen Stammzellen nutzen. Eine RNAi-Selektionskassette ist für die Herstellung von targeting-Konstrukten geeignet, da eine Kopie der Kassette für die Selektion eines über homologe Rekombination veränderten Allels des Zielgens ausreicht. Wird in einem solchen Fall die Selektionskassette mit Erkennungssequenzen wie beispielsweise loxP, frt, attb-attp oder ähnliche für Rekombinasen wie beispielsweise cre, flp, phiC31 oder ähnliche flankiert, könnte sie mittels Transfektion eines Rekombinase-Expressionsvektors wieder in vivo entfernt werde. Sofern in einer bevorzugten Ausführungsform dabei das HPRT-RNAi-Konstrukt verloren geht und die Zellen wieder HPRT exprimieren, können solche Klone mittels HAT-Medium angereichert werden.

Die Erfindung betrifft daher weiterhin ein Verfahren zur Herstellung eines transgenen Säugers, umfassend die Schritte: (a) Injektion der erfindungsgemäßen embryonalen Stammzelle oder einer nach dem erfindungsgemäßen Verfahren selektionierten embryonalen Stammzelle in Blastocysten eines Säugers, (b) Übertragen der Blastocysten in die Gebärmutter eines Säugers, (c) Austragen des transgenen Säugers.

Außerdem betrifft die Erfindung ein Verfahren zur Herstellung eines transgenen Säugers, wobei die erfindungsgemäßen Stammzellen oder nach dem erfindungsgemäßen Verfahren selektionierte embryonale Stammzellen (a) mit Blastomeren aggregiert werden, (b)in die Gebärmutter eines Säugers übertragen werden und (c) der transgene Säuger ausgetragen wird.

Die technische Lehre der vorliegenden Erfindung kann insbesondere genutzt werden um ES-Tiere, einschliesslich ES-Mäuse herzustellen. Dies kann beispielsweise nach dem Verfahren von Schwenk et al., 2003 (Mol. Cell. Biol., 23: 3982-3989) oder nach dem Verfahren von Nagy et al., 1990 (Development 110:815-821) erfolgen. Hierbei werden diploide embryonale Stammzellen (ES), insbesondere die erfindungsgemäßen Zellen oder Zellen die nach dem erfindungsgemäßen Vefahren hergestellt und/oder selektioniert wurden, in eine tetraploide Blastozyste eingebracht, die beispielsweise mittels Elektrofusion befruchteter Eizellen im 2-Zell-Stadium hergestellt werden kann. Das Einbringen der erfindungsgemäßen Zellen kann beispielsweise durch Mikroinjection erfolgen; anschließend werden die Blastocysten in die Gebährmutter von Ammen implantiert.

Weiterhin betrifft die Erfindung ein Verfahren zur Herstellung von transgenen Tieren, wobei der Zellkern von erfindungsgemäßen nichtmenschlichen Zellen in entkemte nichtmenschliche Oozyten transferiert wird.

Des weiteren betrifft die Erfindung ein transgenes Tier, das eine Zelle enthält die nach einem oder mehreren Schritt(en) des erfindungsgemäßen Verfahrens selektioniert wurde, oder das eine erfindungsgemäße Zelle enthält oder das nach dem erfindungsgemäßen Verfahren zur Herstellung eines transgenen Tiers oder zur Herstellung eines transgenen Säugers hergestellt wurde. Bevorzugte transgene Tiere sind Maus, Ratte, Schwein, Rind, Primat, Zebrafisch, C.elegans und Drosopila melanogaster.

Gleichzeitig schließt die Erfindung Tiere ein, in die eine erfindungsgemäße Kassette mittels Microinjektion in den männlichen Vorkern einer befruchteten Eizelle gelangte.

Erfindungsgemäß kann man aus der inneren Zellmasse von Blastocysten (Embryonen etwa am Tag 3,5 der Embryonalentwicklung) pluripotente embryonale Stammzellen (ES-Zellen) isolieren. Diese Zellen werden dann z.B. in vitro mit dem erfindungsgemäßen Konstrukt transfiziert. Nach einer homologen Rekombination in den ES-Zellen, können die ES-Zellen in Blastocysten reinjiziert werden. Die Blastocysten werden in den reproduktiven Trakt einer Amme transferiert und von dieser ausgetragen.

Grundsätzlich können für den Gentransfer in die Zellen virale wie nicht-virale Transfersysteme verwendet werden. Geeignete virale Vektoren umfassen Retrovirus, Adenovirus, Adeno-assoziierten Virus, Herpesvirus, Vaccinia-Virus, Poliovirus und ähnliche. Alternativ können für den Gentransfer nicht-virale Techniken verwendet werden wie z.B. rezeptorvermittelter, gezielter DNA Transfer durch Verwendung von Liganden-DNA-Konjugaten oder Adenovirus-Liganden-DNA-Konjugaten, Lipofektion, Membranfusion oder direkte Mikroinjektion. Die Herstellung transgener Tiere ist dem Fachmann hinreichend bekannt und wird nach üblichen Verfahren durchgeführt (siehe z.B. Hogan, B., Beddington, R., Costantini, F. und Lacy, E. (1994), Manipulating the Mouse-Embryo; A Laboratory Manual, 2. Aufl., Cold Spring Harbor Laboratory, Cold Spring Harbor, NY).

Schließlich betrifft die Erfindung ein Verfahren zur Herstellung einer transgenen Pflanze, umfassend die Schritte: (a) Kultivieren des erfindungsgemäßen Protoplasten oder eines nach dem erfindungsgemäßen Verfahren hergestellten Protoplasten in geeignetem Nährmedium und (b) Regeneration ganzer Pflanzen.

Die erfindungsgemäßen Pflanzenzellen können in den erfindungsgemäßen Verfahren zur Regeneration ganzer, intakter Pflanzen verwendet werden. Gegenstand der vorliegenden Erfindung sind somit auch transgene Pflanzen, die erhältlich sind durch Regeneration einer erfindungsgemäßen Pflanzenzelle, sowie Pflanzen, die erfindungsgemässe transgene Pflanzenzellen enthalten. Dem Fachmann sind zahlreiche Verfahren und Nährmedien zur Kultivierung der Pflanzenzellen und Protoplasten bekannt, die in den erfindungsgemäßen Verfahren verwendet werden können. Die Auswahl der Kulturbedingungen richtet sich jeweils nach der betreffenden Zelle.

Die Figuren zeigen:

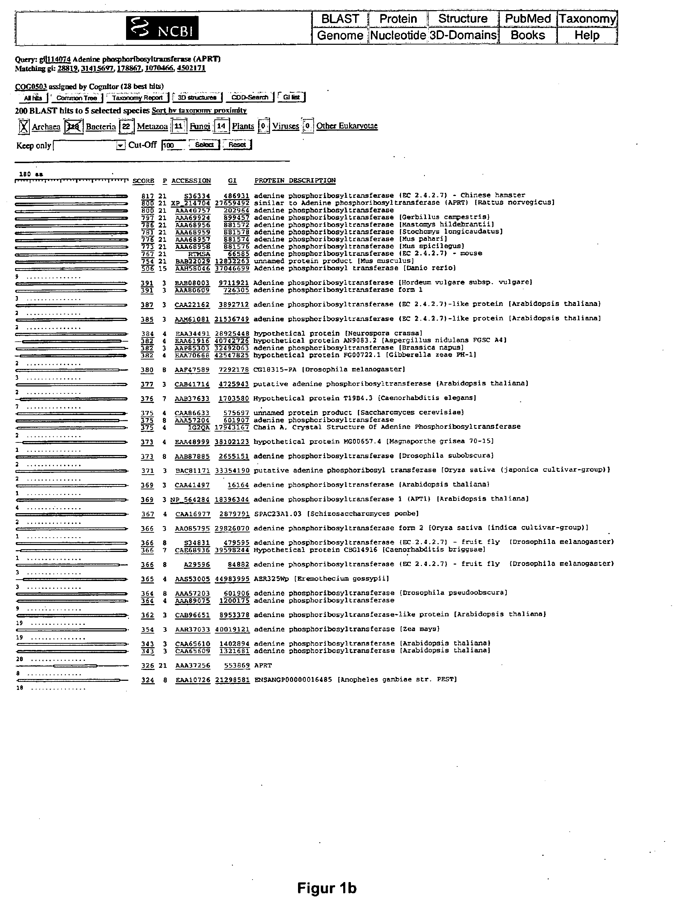

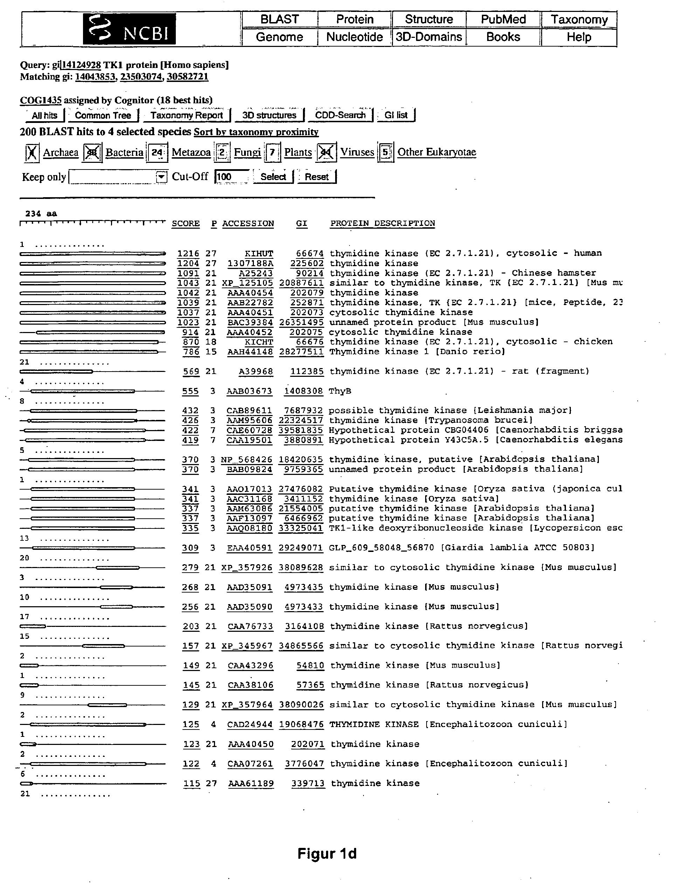

- Figur 1 a-d:: HomoloGene-Datenbankeinträge des NCBI. Die Figuren a-d zeigen die Datenbankeinträge HomoloGene build 36 (last update 05/25/2004) der Gene: HPRT HomoloGene: 162, APRT Homologene: 413, Thymidine Kinase HomoloGene: 2446, DHFR HomoloGene: 619.

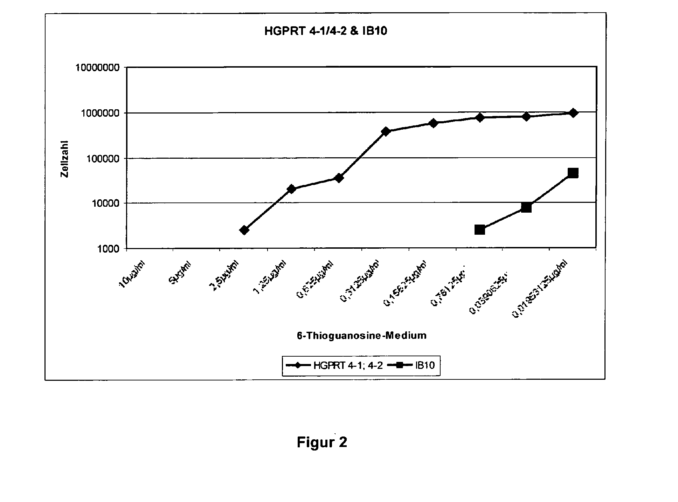

- Figur 2:: Stabil HPRT 4-1/4-2 transfizierte IB10 ES-Zellen entwickeln Resistenz gegen 6-Thioguanosin. Zur Untersuchung der Wirkung des HPRTRNAi-Konstruktes 4-1/4-2 wurden jeweils 10⁴ Zellen eines Klons pro Loch einer 24-Loch-Zellkulturplatte ausgesät und in ES-Medium mit unterschiedlichen Konzentrationen von 6-Thioguanosin für acht Tage weiterkultiviert. Die Auswertung erfolgte durch Bestimmung der Zellzahl nach acht Tagen. Nichttransfizierte IB-10 ES-Zellen wurden als Kontrolle den gleichen Selektionsbedingungen ausgesetzt.

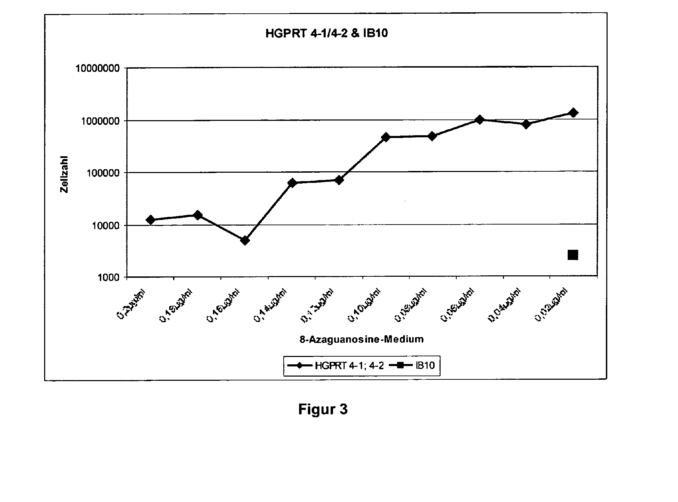

- Figur 3:: Stabil HPRT 4-1/4-2 transfizierte IB10 ES-Zellen entwickeln Resistenz gegen 8-Thioguanosin. Zur Untersuchung der Wirkung des HPRTRNAi-Konstruktes 4-1/4-2 wurden jeweils 10⁴ Zellen eines Klons pro Loch einer 24-Loch-Zellkulturplatte ausgesät und in ES-Medium mit unterschiedlichen Konzentrationen von 8-Azaguanosin für acht Tage weiterkultiviert. Die Auswertung erfolgte durch Bestimmung der Zellzahl nach acht Tagen. Nichttransfizierte IB-10 ES-Zellen wurden als Kontrolle den gleichen Selektionsbedingungen ausgesetzt.

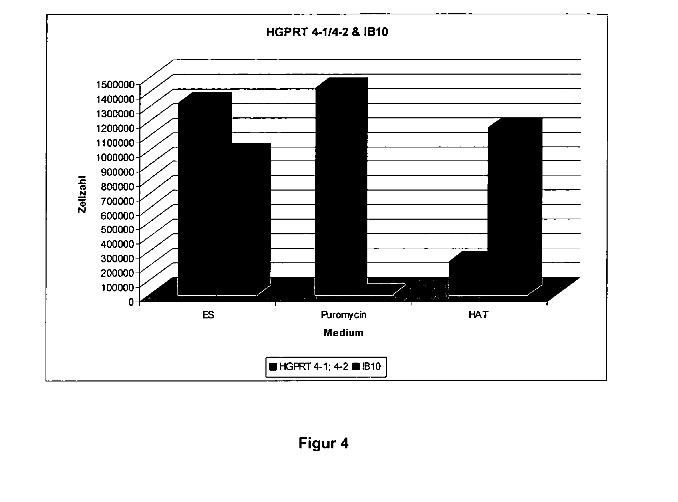

- Figur 4:: Stabil HPRT 4-1/4-2 transfizierte IB10 ES-Zellen werden sensitiv für HAT-Medium. Zur Untersuchung der Wirkung des HPRT-RNAi-Konstruktes 4-1/4-2 wurden jeweils 10⁴ Zellen eines Klons pro Loch einer 24-Loch-Zellkulturplatte ausgesät und in ES-Medium ohne bzw. mit Zusatz von Puromycin oder HAT für acht Tage weiterkultiviert. Die Auswertung erfolgte durch Bestimmung der Zellzahl nach acht Tagen. Nichttransfizierte IB-10 ES-Zellen wurden als Kontrolle den gleichen Selektionsbedingungen ausgesetzt.

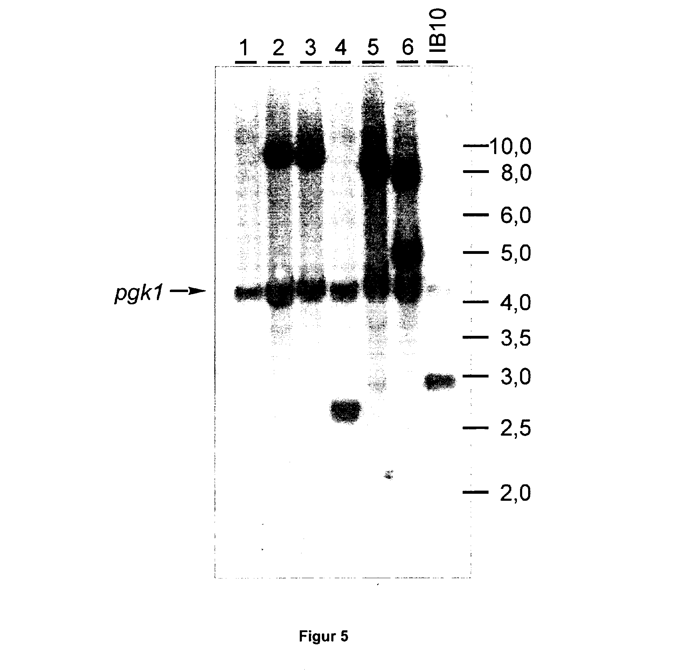

- Figur 5:: Bestimmung der HPRT-RNAi-Kopienzahl in stabil transfizierten IB10 ES-Zellen: Zur Bestimmung der HPRT-RNAi-Kopienzahl wurde genomische DNA von stabil mit dem Konstrukt 4-1/4-2 transfizierten IB10-ES-Zellklonen (Spuren 1-6) mit HindIII verdaut, elektrophoretisch aufgetrennt, geblottet und mit einem 1040bp langen Hindlll/Sacll-Fragment aus dem HPRT-RNAi-Vektor 4-1/4-2 hybridisiert, das neben dem H1-Promotor und den HPRT-Sequenzen auch den pGK-Promotor des Puromycin-Resistenzgens beinhaltet. Als Kontrolle diente genomische DNA von nicht-transfizierten IB10-Zellen (IB10). pgk1 kennzeichnet das Fragment, das durch die Hybridisierung mit dem endogenen pgk1-Gen hervorgerufern wird.

Die Beispiele erläutern die Erfindung:

### Beispiel 1: Klonierung von gegen murine HPRT mRNA gerichteten siRNA-Konstrukten

Entsprechend des von Tuschl und Mitarbeitern für synthetische doppelsträngige shRNA-Molekülen entwickelten Algorithmus (Elbashir et al. 2001) wurde eine gegen die murine HPRT-mRNA gerichtete Sequenz identifiziert.

Diese wurde als indirekter repeat in einem sense- und anti-sense-Oligonukleotid synthetisiert (4-1 und 4-2), wobei die beiden Hälften des repeats durch einen Sequenzabschnitt getrennt wurden, der in der mRNA einen hairpin loop bildet. An den Enden wurden an beiden Strängen jeweils Nucleotide zur Erzeugung eines BamHI-Überhanges am 5'-Ende und eines HindIII-Überhanges am 3'-Ende hinzugefügt. Zur Erzeugung doppelsträngiger DNA-Fragmente mit den entsprechenden Überhängen wurden jeweils die beiden einzelsträngigen Oligonucleotide in äquimolarem Verhältnis gemischt, für 3 min auf 95°C erhitzt und dann in ein 60°C warmes Wasserbad überführt, das langsam auf Raumtemperatur abgekühlt wurde. Die beiden so erzeugten doppelsträngige DNA-Fragment wurde anschließend jeweils in den BglII/HindIII geschnittenen Vektor pSuper-retro ligiert und in E.coli Dh5alpha transformiert. Durch Restriktionsverdaus von Plasmid DNA aus entsprechenden Transformanden wurden Plasmide die das gegen HPRT gerichtete Fragment enthielten identifiziert. Durch Sequenzierung wurde die Identität der Konstrukte verifiziert, das im folgenden dargestellte Konstrukt wird als 4-1/4-2 bezeichnet.

### Beispiel 2: Kultur und Transfektion von IB10 ES-Zellen

Murine embryonale Stammzellen wurden auf gelatine-beschichteten Zellkulturschalen ohne feeder Zellen unter Standard-Bedingungen (Torres und Kühn, 1997) gehalten. Für die Transfektion wurden 10µg des transfizierenden HPRT-RNAi-Konstrukts 4-1/4-2 mittels HindIII linearisiert und anschließend aufgereinigt. Pro Versuch wurden 107 Zellen mit 10µg Plasmid-DNA mittels Elektroporation transfiziert. Anschließend wurden die Zellen auf gelatine-beschichteten Zellkulturschalen ausgesät und zwei Tage in normalem ES-Medium gehalten. Am dritten Tag nach der Transfektion wurde das Medium gegen Selektionsmedium (ES-Medium mit 5µg/ml Puromycin) ausgetauscht, in dem nur stabil transfizierte Zellen überleben sollten. Sich entwickelnde Klone wurden nach acht Tagen isoliert, einzeln weiter kultiviert, expandiert und eingefroren.

### Beispiel 3: HPRT-abhängige Selektion

Zur Untersuchung der Wirkung des HPRT-RNAi-Konstruktes wurden jeweils 104 Zellen eines Klons pro Loch einer 24-Loch-Zellkulturplatte ausgesät und in unterschiedlichen Konzentrationen von HAT-ES-Medium, ES-Medium mit 6-Thioguanosin sowie ES-Medium mit 8-Azaguanosin für acht Tage weiterkultiviert. Als Kontrollen dienten Kulturen in ES-Medium ohne Selektionszusätze und ES-Medium mit 5µg/ml Puromycin. Außerdem wurden nichttransfizierte IB-10 ES-Zellen zur Kontrolle den gleichen Selektionsbedingungen ausgesetzt. Die Auswertung erfolgte durch Bestimmung der Zellzahl nach acht Tagen.

Nach Transfektion von HPRT-RNAi-Vektoren in IB10 ES-Zellen wurden puromycinresistente Klone erhalten. Das hier beispielhaft beschriebene Konstrukt (4-1/4-2) entwickelte Resistenz gegen 6-Thioguanosin, die mindestens 10-fach höher ist als die von IB-10-Zellen (Figur 2). Der gleiche Unterschied wurde mit 8-Azaguanosin beobachtet (Figur 3). Gleichzeitig waren die Zellen sensitiv gegenüber HAT-Medium und wuchsen 6-fach schlechter in diesem Medium als Wildtyp-Zellen (Figur 4). Die Zellen verhalten sich in diesem Fall also so, wie dies von IB-10 ohne funktionelles HPRT-Gen zu erwarten wäre.

### Beispiel 4: Bestimmung der HPRT-RNAi-Kopienzahl

Um auszuschließen, daß die bei der Selektion beobachteten Effekte nicht auf unterschiedlichen Kopienzahlen der in die Genome der Klone integrierten HPRT-RNAi (des Konstruktes 4-1/4-2) beruhen, wurde die Anzahl der integrierten Kopien mittels Southern-blotting bestimmt. Dazu wurde aus den Klonen isolierte genomische DNA mit HindIII verdaut, auf einem Agarosegel elektrophoretisch aufgetrennt und unter alkalischen Bedingungen auf eine Nylonmembran transferiert. Nach Neutralisieung wurde die DNA mit einem 1040bp langen HindIII/SacII-Fragment des HPRT-RNAi-Vektors hybridisiert, das neben dem H1-Promotor und den HPRT-Sequenzen auch den pGK-Promotor des Puromycin-Resistenzgens beinhaltet. Deshalb hybridisiert die Probe auch mit dem endogenen pGK1-Gen und die resultierende Bande kann zur Quantifizierung der durch das jeweilige Transgen erzeugten Banden herangezogen werden.

Bei den untersuchten Klonen trat jeweils nur eine konstruktspezifische Bande auf, welche die gleiche Intensität aufwies wie die durch das endogene X-chromosomale pGK1-Gen hervorgerufene Bande (Figur 5). IB-10 besitzen aufgrund des männlichen Genotyps der Zelle nur eine pGK1-Kopie. Die untersuchten Klone trugen also jeweils auch nur eine Kopie des HPRT-RNAi-Konstruktes. Offensichtlich ist also eine Kopie des HPRT-RNAi-Konstruktes ausreichend die Funktion des endogenen HPRT-Genes soweit zu inaktivieren, daß sie eine Selektion mittels 6-Thioguanosin und 8-Azaguanosin möglich ist sowie eine negative Selektion mittels HAT-Medium ermöglicht.

## Patentansprüche

1. Verfahren zur Herstellung einer durch Inaktivierung oder Reduzierung einer endogenen Genfunktion selektionierbaren eukaryontischen Zelle, umfassend die Schritte
(a) der Einführung einer oder mehrerer Vektoren in die Zelle und
(b) der Expression einer von dem einen oder den mehreren Vektoren kodierten siRNA und vorzugsweise shRNA, die gegen ein endogenes selektionierbares Gen gerichtet ist und dieses inaktiviert, wobei die siRNA oder shRNA das Transkriptionsprodukt einer RNAi-Selektionskassette ist, wobei die Selektionskassette einen zumindest 19 Nukleotide langen Abschnitt der transkribierten Region des Gens umfasst, der funktionell mit einem Promoter und einem Transkriptionsterminationssignal verbunden ist.

2. Verfahren nach Anspruch 1, umfassend den weiteren Schritt (c) der Expression einer Rekombinase, wobei die RNAi-Selektionskassette nach homologer Rekombination gebildet wird, wobei vor der Rekombination zwischen den Rekombinationsvorstufen, zwischen Promotor und RNAi-Selektionskassette oder innerhalb der RNAi-Selektionskassette eine 5' und eine 3' Rekombinaseerkennungssequenz und zwischen diesen eine trennende Nukleotidsequenz angeordnet ist, wobei die trennende Nukleotidsequenz ein Transkriptionsterminationssignal enthält und wobei durch die Expression der Rekombinase eine homologe Rekombination an den Rekombinaseerkennungssequenzen erfolgt.

3. Verfahren nach Anspruch 2, wobei die Rekombinaseerkennungssequenzen zwischen der ersten und zweiten Sequenz der invertierten Sequenzwiederholung der shRNA angeordnet sind.

4. Verfahren nach Anspruch 3, wobei
(i) der Promoter, die 5' Rekombinaseerkennungssequenz und die erste Sequenz der invertierten Sequenzwiederholung der shRNA und
(ii) die 3' Rekombinaseerkennungssequenz und die zweite Sequenz der invertierten Sequenzwiederholung der shRNA
auf unterschiedlichen Vektoren angeordnet sind.

5. Verfahren nach einem der Ansprüche 1 bis 4, umfassend den weiteren Schritt (d) des Kultivierung und Anreicherung von Zellen die die RNAi-Selektionskassette enthalten und exprimieren oder (d') Kultivierung und Anreicherung von Zellen die die RNAi-Selektionskassette nicht enthalten und exprimieren.

6. Verfahren nach einem der Ansprüche 1 bis 5, wobei das selektionierbare Gen positiv und/oder negativ selektionierbar ist.

7. Verfahren nach einem der Ansprüche 1 bis 6, wobei das endogene selektionierbare Gen ein Produkt mit der Fähigkeit kodiert, eine nicht selektierende Vorstufe einer Substanz A in ein Produkt B mit selektionierenden oder selektionierbaren Eigenschaften umzuwandeln und die Zelle in Gegenwart der Substanz A kultiviert wird.

8. Verfahren nach einem der Ansprüche 1 bis 7, wobei die nicht selektierende Substanz A eine ungiftige Vorstufe eines Toxins und das Produkt B ein Toxin ist.

9. Verfahren nach Anspruch 7 oder 8, wobei das Gen APRT, DHFR oder TK ist.

10. Verfahren nach Anspruch 7 oder 8, wobei das Gen HPRT ist.

11. Verfahren nach Anspruch 10, wobei 6-Thioguanosin oder 8-Azaguanosin zur Selektion verwendet wird.

12. Verfahren nach einem der Ansprüche 1 bis 11, wobei zusammen mit der RNAi-Selektionskassette oder deren Rekombinationsvorstufe, oder nachfolgend, zumindest eine weitere Nukleotidsequenz in die Zelle eingebracht wird.

13. Verfahren nach Anspruch 12, wobei die zumindest eine weitere Nukleotidsequenz zwei Nukleotidsequenzen mit Homologie zu zellulären genomischen Nukleotidsequenzen sind, die die RNAi-Selektionskassette oder deren Rekombinationsvorstufen an deren 5' und 3' Ende flankieren.

14. Verfahren nach Anspruch 12, wobei die weitere in die Zelle eingebrachte Nukleotidsequenz auf dem Vektor gelegen ist, der die RNAᵢ-Selektionskassette enthält und die weitere Nukleotidsequenz von den 5' und 3' flankierenden Sequenzen mit Homologie zu zellulären Nukleotidsequenzen umfasst wird.

15. Verfahren nach Anspruch 12, wobei die weitere in die Zelle eingebrachte Nukleotidsequenz auf einem separaten Vektor gelegen ist und 5' und 3' der weiteren Nukleotidsequenz Nukleotidsequenzen mit Homologie zu zellulären genomischen Nukleotidsequenzen vorhanden sind.

16. Verfahren nach Anspruch 14 oder 15, wobei die weitere in die Zelle eingebrachte Nukleotidsequenz nachfolgend in die Zelle eingebracht wird und 5' und 3' von homologen genomischen Nukleotidsequenzen flankiert wird, die eine Rekombination der weiteren Nukleotidsequenz in die chromosomale Position der RNAᵢ-Selektionskassette ermöglichen.

17. Verfahren nach einem der Ansprüche 12 bis 16, wobei die RNAi-Selektionskassette und/oder die weitere in die Zelle eingebrachte Nukleotidsequenz zusätzlich von Rekombinase-Erkennungssequenzen flankiert wird.

18. Verfahren nach einem der Ansprüche 12 bis 17, wobei die weitere in die Zelle eingebrachte Nukleotidsequenz ein Gen oder ein Fragment davon ist.

19. Verfahren nach Anspruch 19, wobei das Gen eine Mutante oder allelische Variante eines endogenen Gens der Zelle ist.

20. Verfahren nach Anspruch 19, wobei das Gen ein exogenes Gen der Zelle ist.

21. Verfahren nach einem der Ansprüche 1 bis 20, ferner umfassend die folgenden Schritte:
(e) eine Inaktivierung oder Reduktion der RNAᵢ-Expression; und
(f) eine Selektion und Anreicherung von Zellen, die keine RNAi-basierte Inaktivierung oder Reduktion des endogenen selektionierbaren Gens aufweisen.

22. Verfahren nach Anspruch 21, wobei die Inaktivierung der RNAi Expression eine Rekombinase-vermittelte Deletion ist und den Schritt der Expression einer Rekombinase umfasst.

23. Verfahren nach Anspruch 21 oder 22, wobei das endogene selektionierbare Gen HPRT ist und die Selektion von Zellen, die keine RNAi-basierte Inaktivierung des endogenen selektionierbaren Gens aufweisen in Gegenwart von HAT-Medium erfolgt.

24. Verfahren nach einem der Ansprüche 13 bis 23, wobei der Vektor eine weitere Kassette enthält, die bei zufälliger Integration in ein zelluläres Gen zur Inaktivierung desselben führt.

25. Verfahren nach einem der Ansprüche 1 bis 24, wobei die eukaryontische Zelle eine Pflanzenzelle ist.

26. Verfahren nach Anspruch 25, wobei die Pflanzenzelle ein Protoplast ist.

27. Verfahren nach einem der Ansprüche 1 bis 24, wobei die eukaryontische Zelle eine Vertebratenzelle oder Invertebratenzelle ist.

28. Verfahren nach Anspruch 27, wobei die Vertebratenzelle eine Säugerzelle ist.

29. Verfahren nach Anspruch 28, wobei die Säugerzelle eine Zelle ist die ausgewählt wird aus Mensch, Maus, Ratte, Schwein, Rind und Primat.

30. Verfahren nach Anspruch 27, wobei die Zelle eine embryonale Stammzelle, eine adulte Stammzelle, eine hematopoietische Stammzelle, Körperzelle oder eine Zelle einer etablierten Zelllinie ist.

31. Eukaryontische Zelle, umfassend eine RNAi-Selektionskassette die gegen ein endogenes selektionierbares Gen gerichtet ist, und dessen Funktion inaktiviert; wobei die RNAi-Selektionskassette einen zumindest 19 Nukleotide langen Abschnitt des Gens umfasst, der funktionell mit einem Promotor und einem Transkriptionsterminationssignal verbunden ist.

32. Zelle nach Anspruch 31, wobei das endogene selektionierbare Gen ausgewählt wird aus der Gruppe bestehend aus HPRT, APRT, DHFR oder TK.

33. Zelle nach Anspruch 31 oder 32, wobei die RNAi-Selektionskassette stabil in das Genom der Zelle integriert ist.

34. Zelle nach einem der Ansprüche 31 bis 33, wobei die Zelle eine Pflanzenzelle ist.

35. Zelle nach Anspruch 34, wobei die Pflanzenzelle ein Protoplast ist.

36. Transgene Pflanze die die Pflanzenzelle nach Anspruch 34 oder 35 enthält.

37. Zelle nach einem der Ansprüche 31 bis 33, wobei die Zelle eine Vertebratenzelle oder Invertebratenzelle ist.

38. Zelle nach Anspruch 37, wobei die Vertebratenzelle eine Säugerzelle ist.

39. Zelle nach Anspruch 38, wobei die Säugerzelle eine Zelle ist die ausgewählt wird aus der Gruppe bestehend aus Mensch, Maus, Ratte, Schwein, Rind und Primat.

40. Zelle nach Anspruch 39, wobei die Zelle eine embryonale Stammzelle, eine adulte Stammzelle, eine hematopoietische Stammzelle, Körperzelle oder eine etablierte Zellinie ist.

41. Verfahren zur Herstellung eines transgenen Tieres, wobei
a) Zellen nach einem oder mehreren der in den Ansprüchen 1 bis 30 genannten Verfahrensschritte behandelt werden und
b) aus den Zellen ein lebensfähiger Organismus gezogen wird.

42. Verfahren zur Herstellung eines transgenen Säugers, umfassend die Schritte:
(a) Injektion der embryonalen Stammzelle aus Anspruch 40 oder einer nach dem Verfahren von Anspruch 30 selektionierten embryonalen Stammzelle in Blastocysten eines Säugers,
(b) Übertragen der Blastocysten in die Gebärmutter eines Säugers, und
(c) Austragen des transgenen Säugers.

43. Transgenes Tier, das eine Zelle enthält die nach einem oder mehreren der in den Ansprüchen 1 bis 30 genannten Verfahrensschritte selektioniert wurde, oder das eine Zelle nach Anspruch 31 bis 33 oder 37 bis 40 enthält oder das nach dem Verfahren von Anspruch 41 oder 42 hergestellt wurde.

44. Verfahren zur Herstellung einer transgenen Pflanze, umfassend die Schritte:
(a) Kultivieren des Protoplasten aus Anspruch 35 oder eines nach dem Verfahren von Anspruch 26 hergestellten Protoplasten in geeignetem Nährmedium, und
(b) Regeneration ganzer Pflanzen.
